# EUROPEAN PATENT SPECIFICATION

(11) **EP 4 041 240 B1**
(45) Date of publication and mention of the grant of the patent: **24.06.2026**
(21) Application number: 20797943.6
(22) Date of filing: 08.10.2020
(51) Int. Cl.: A61K 31/519, A61K 31/573, A61P 17/00, A61P 37/00

(54) **METHODS OF TREATING PEMPHIGUS BY ADMINISTERING (R)-2-[3-[4-AMINO-3-(2-FLUORO-4-PHENOXY-PHENYL)PYRAZOLO[3,4-D]PYRIMIDIN-1-YL]PIPERIDINE-1-CARBONYL]-4-METHYL-4-[4-(OXETAN-3-YL)PIPERAZIN-1-YL]PENT-2-ENENITRILE**
VERFAHREN ZUR BEHANDLUNG VON PEMPHIGUS DURCH VERABREICHUNG VON (R)-2-[3-[4-AMINO-3-(2-FLUORO-4-PHENOXY-PHENYL)PYRAZOLO[3,4-D]PYRIMIDIN-1-YL]PIPERIDIN-1-CARBONYL]-4-METHYL-4-[4-(OXETAN-3-YL)PIPERAZIN-1-YL]PENT-2-ENENITRIL
PROCÉDÉS DE TRAITEMENT DU PEMPHIGUS PAR ADMINISTRATION DE (R)-2-[3-[4-AMINO-3-(2-FLUORO-4-PHÉNOXY-PHÉNYL)PYRAZOLO[3,4-D]PYRIMIDIN-1-YL]PIPÉRIDINE-1-CARBONYL]-4-MÉTHYL-4-[4-(OXÉTAN-3-YL)PIPÉRAZIN-1-YL]PENT-2-ÈNE-NITRILE

(30) Priority: 09.10.2019 US 201962913029 P; 03.12.2019 US 201962942877 P
(43) Date of publication of application: 17.08.2022
(73) Proprietor: Principia Biopharma Inc., Morristown, NJ 07960 (US)
(72) Inventor: NEALE, Ann, San Francisco, California 94080 (US); THOMAS, Dolca, San Francisco, California 94080 (US)
(74) Representative: J A Kemp LLP
(86) International application number: PCT/US2020/054809
(87) International publication number: WO 2021/072095

(56) References cited:
- WO-A1-2015/127310
- WO-A1-2016/100914
- WO-A1-2019/208805
- KENTARO IZUMI ET AL: "Current Clinical Trials in Pemphigus and Pemphigoid", FRONTIERS IN IMMUNOLOGY, vol. 10, 3 May 2019 (2019-05-03), pages 978, XP055761713, DOI: 10.3389/fimmu.2019.00978
- DF MURRELL ET AL: "A Pilot Study of the Efficacy of a Bruton's Tyrosine Kinase Inhibitor in the Treatment of Dogs with Pemphigus Foliaceus", AUSTRALASIAN JOURNAL OF DERMATOLOGY, vol. 58, no. S1, 1 January 2017 (2017-01-01), pages 73, XP055649827
- VAN BEEK N ET AL: "Therapy of pemphigus", HAUTARZT, SPRINGER VERLAG, BERLIN, DE, vol. 70, no. 4, 18 March 2019 (2019-03-18), pages 243 - 253, XP036750048, ISSN: 0017-8470, [retrieved on 20190318], DOI: 10.1007/S00105-019-4385-9
- PRINCIPIA BIOPHARMA: "A Study of PRN1008 in Adult Patients With Pemphigus Vulgaris", CLINICALTRIALS.GOV, 10 March 2016 (2016-03-10), XP055764616, Retrieved from the Internet <URL:https://clinicaltrials.gov/ct2/show/study/NCT02704429> [retrieved on 20210113]
- PATRICK F. SMITH ET AL: "A phase I trial of PRN1008, a novel reversible covalent inhibitor of Bruton's tyrosine kinase, in healthy volunteers : A phase I study of PRN1008", BRITISH JOURNAL OF CLINICAL PHARMACOLOGY., vol. 83, no. 11, 1 August 2017 (2017-08-01), GB, pages 2367 - 2376, XP055764604, ISSN: 0306-5251, DOI: 10.1111/bcp.13351

## Description

The present invention relates to a compound which is (R)-2-[3-[4-amino-3-(2-fluoro-4-phenoxy-phenyl)pyrazolo[3,4-d]pyrimidin-1-yl]piperidine-1-carbonyl]-4-methyl-4-[4-(oxetan-3-yl)piperazin-1-yl]pent-2-enenitrile or a pharmaceutically acceptable salt thereof, for use in treating pemphigus, wherein said compound is administered to a human patient twice a day at a dose of 400 mg for at least 14 days, and wherein the human patient is characterized by relapsing pemphigus prior to the administration of said compound. Also disclosed herein is said compound for use in methods for achieving certain outcomes in a human patient population undergoing treatment for pemphigus comprising administering to each member of the human patient population said compound.

Bruton's agammaglobulinemia tyrosine kinase (BTK) is an essential signaling element downstream of the B-cell receptor (BCR), Fc-gamma receptor (FcyR), and Fc-epsilon receptor (FcεR). BTK is a non-receptor tyrosine kinase and a member of the TEC family of kinases. BTK is essential to B cell lineage maturation, and inhibition of BTK activity in cells produces phenotypic changes consistent with blockade of the BCR. Illustratively, BTK inhibition results in the down-regulation of various B-cell activities, including cell proliferation, differentiation, maturation, and survival, and the up-regulation of apoptosis.

Rather than acting in an "on/off switch" manner, BTK may be best viewed as an immune function "modulator" (Crofford LJ et al., 2016; Pal Singh S et al., 2018). Important insights into BTK function come from loss of function analyses in humans and mice. Individuals with loss of function mutations in the BTK gene develop X-linked agammaglobulinemia (XLA), characterized by a complete absence of circulating B cells and plasma cells, and very low levels of immunoglobulins of all classes (Tsukada 1993, Vetrie 1993). This indicates the potential for BTK inhibition to suppress production of autoantibodies thought to be important in development of autoimmune diseases, such as, e.g., pemphigus vulgaris (PV).

While BTK is not expressed in T cells, natural killer cells, and plasma cells and has no traceable direct functions in T cells and plasma cells (Sideras and Smith 1995; Mohamed et al., 2009), the enzyme also regulates the activation of other hematopoietic cells, such as basophils, mast cells, macrophages, neutrophils, and platelets. For example, BTK plays a role in the activation of neutrophils, which are key players in the inflammatory response that contributes to wound healing but may also cause tissue damage (Volmering S et al., 2016).

Accordingly, a selective BTK inhibitor has the potential to target multiple pathways involved in inflammation and autoimmunity, including modulating BCR-mediated B-cell pathways and inhibiting FcyR-induced cytokine release from monocytes and macrophages, FcεR-induced mast cell degranulation, granulocyte migration, and mediator release. Based on these effects, a selective BTK inhibitor may block the initiation and progression of various inflammatory diseases and mitigate tissue damage resulting from these diseases. Although individuals with loss of function mutations in the BTK gene have decreased humoral immunity and are susceptible to pyogenic bacterial and enterovirus infections, requiring treatment with intravenous immunoglobulin, inhibition of BTK in individuals with an intact immune system is not predicted to produce similar susceptibility to infection.

Pemphigus is a rare B cell-mediated autoimmune disease that causes debilitating intraepithelial blisters and erosions on the skin and/or mucous membranes. The characteristic intraepidermal blisters observed in pemphigus patients result from the binding of IgG autoantibodies to certain keratinocyte desmosomal adhesion proteins, desmogleins 1 and 3 (Dsg1 and Dsg3), resulting in loss of cell adhesion (Amagai M et al., 2012; Diaz LA et al., 2000). For example, PV is driven by autoantibodies to epidermal proteins.

Pemphigus affects approximately 0.1-0.5/100,000 people each year and carries a 10% mortality rate, generally due to infections arising from compromised tissues and treatment side effects (Scully et al., 2002; Scully et al., 1999). Because pemphigus is a chronic disease for which there is no cure, most pemphigus patients are existing cases. The current standard of care for new onset pemphigus is high-dose corticosteroids (CS) (0.5-1.5 mg/kg/day) (Murrell DF et al., 2018), alone or in combination with other immunosuppressant drugs such as rituximab, mycophenolate mofetil, or azathioprine (Kasperkiewicz M et al., 2017). Illustratively, PV is responsive acutely to the anti-inflammatory effects of corticosteroids and within 5 to 35 weeks to B-cell depletion by anti-CD20 therapy (Horvath et al. 2012).

Rituximab, a chimeric monoclonal antibody against the B cell surface marker CD20, was recently approved by the FDA and the European Medicines Agency (EMA) for the treatment of moderate to severe pemphigus vulgaris (PV) based on studies done in newly diagnosed patients with improved rates of steroid/treatment-free complete remission (CR) compared to CS alone (Joly P et al., 2017; RITUXAN Prescribing Information; Cianchini et al., 2007). However, patients treated with rituximab still required moderate to high steroid doses (0.5-1.0 mg/kg/day) for two to three months before steroid tapering could be initiated, and no randomized controlled studies have assessed the efficacy of rituximab in relapsing patients or patients with pemphigus foliaceus (PF). Other pemphigus treatments like intravenous immunoglobulin (IVIG), plasmapheresis, and extracorporeal photopheresis (Harman KE et al., 2017) have some benefits but are yet to be evaluated in randomized controlled trials or large patient populations (Amagai M et al., 2009; Martin LK et al., 2009). Therefore, fast-acting, steroid-sparing, and conveniently administered immunomodulatory therapies with improved safety profiles are greatly needed.

B cells play key roles in the production of autoantibodies involved in pemphigus pathology and in cellular tolerance mechanisms, thus presenting as an attractive target for pemphigus treatment. For example, BTK inhibition is an appealing therapeutic strategy for pemphigus.

Several orally administered BTK inhibitors (BTKi), including ibrutinib (PCI-32765) and spebrutinib (CC-292), are currently marketed or in clinical development for a range of indications (Lee A et al., 2017). For example, ibrutinib has provided further clinical validation of the BTK target and was recently approved for human use in mantle cell lymphoma, Waldenström's macroglobulinemia, and chronic lymphocytic leukemia by the U.S. Food and Drug Administration (FDA), and has also demonstrated activity in other hematological malignancies (Wang 2013, Byrd 2013, Imbruvica Package Insert, 2015). In addition, CC-292 has been reported to be well tolerated in a healthy volunteer population at doses which provide 100% occupancy of the BTK enzyme (Evans 2013). Furthermore, evobrutinib recently demonstrated efficacy for multiple sclerosis in a Phase 2 trial (Montalban X et al., 2019). Other BTKi compounds are in clinical development for various immune-mediated disorders, such as immune thrombocytopenia (NCT03395210), rheumatoid arthritis (NCT03823378, NCT03682705, NCT03233230), and asthma (NCT03944707) (Montalban X et al., 2019; Norman P 2016; Tam CS et al., 2018; Crawford JJ et al., 2018; Min TK et al., 2019; Gillooly KM 2017; Nadeem A et al., 2019).

While covalent BTKi, such as ibrutinib and acalabrutinib, improved on the selectivity issues that plagued many first-generation kinase inhibitors, these inhibitors are typically irreversible, causing permanent modification of both on and off target kinases and side effects such as thrombocytopenia, anemia, platelet aggregation, and hepatotoxicity (RITUXAN Prescribing Information, 2018; Drug Record Kinase Inhibitors, 2019; Khan Y et al., 2019; Paydas S, 2019; IMBRUVICA, 2013; Rigg RA et al., 2016; Tang CPS et al., 2018). Thus, there is a need for treatment modalities for immune-mediated diseases, such as, e.g., pemphigus, based on BTKi with reduced side effects.

Compound **(I)** is a BTK inhibitor of the following structure: 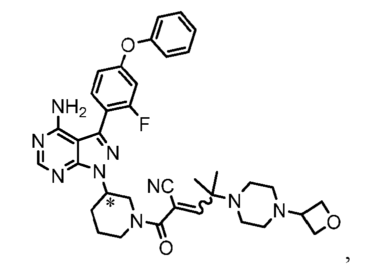 where *C is a stereochemical center. See PCT Publication No. WO 2014/039899, e.g., Example 31.

(R)-2-[3-[4-amino-3-(2-fluoro-4-phenoxy-phenyl)pyrazolo[3,4-d]pyrimidin-1-yl]piperidine-1-carbonyl]-4-methyl-4-[4-(oxetan-3-yl)piperazin-1-yl]pent-2-enenitrile, having the following structure: 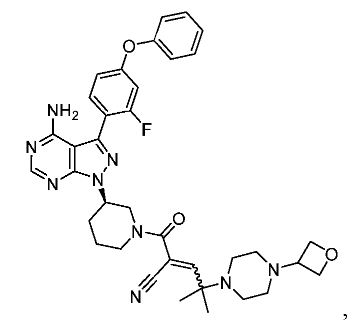 is also known as PRN1008 and rilzabrutinib. This compound has been disclosed in several patent publications, such as, e.g., PCT Publication Nos. WO2014/039899, WO2015/127310, WO2016/100914, WO 2016/105531, and WO2018/005849.

PRN1008 is a novel, highly selective, small molecule inhibitor of non-T cell white blood cell signaling via B-cell receptor, FCyR, and/or FcεR signaling of the BTK pathway. PRN1008 functions as a reversible covalent BTK inhibitor and forms both a non-covalent and a covalent bond with its target, allowing for enhanced selectivity and extended inhibition. In comparison to first and second generation BTKi, PRN1008 has shown minimal cross-reactivity with other molecules and is low risk for off-target effects (Smith PF et al., 2017). Importantly, PRN1008's reversible binding minimizes the likelihood of permanently modified peptides (Serafimova IM 2012).

PRN1008 has shown encouraging results for treatment of immune-mediated diseases. PRN1008 is the most advanced BTKi in development for an autoimmune disease (Phase 3, NCT03762265) and the first BTKi to be evaluated in the treatment of pemphigus. In Phase 1 studies of PRN1008 with 114 healthy volunteers, target BTK occupancy levels were safely and consistently exceeded, suggesting PRN1008 may be highly effective in human pemphigus and other autoimmune diseases. Moreover, preclinical and clinical PK/PD data showed that treatment effects endured even after the compound was cleared from circulation, consistent with an extended target residence time (Hill R et al., 2015) and high occupancy rate (>90% within four hours) (Smith PF et al., 2015.)

PRN1008 has also demonstrated a favorable safety profile. Based on preclinical reproductive toxicity studies, PRN1008 is not expected to harm fetal development or male fertility. In a Phase 1 study in healthy volunteers, the most commonly reported adverse events were gastrointestinal adverse events, including nausea/vomiting and diarrhea. No serious adverse events or deaths were reported, and no participants discontinued treatment due to an adverse event (Smith PF 2017).

As of January 18, 2018, PRN1008 had been administered to 21 patients with pemphigus (pemphigus vulgaris (PV) and pemphigus foliaceus (PF)), 18 of whom had 4 or more weeks of treatment with PRN1008. PRN1008 was well-tolerated in this study. Of the 18 patients with efficacy data, 11 (61%) met the primary endpoint of "Control of Disease Activity" (CDA) on a corticosteroid (CS) dose of ≤ 0.5 mg/kg/day (low-dose CS) by the Week 5 visit. Three patients achieved CDA on no CS. Two patients required high-dose CS temporarily during PRN1008 treatment due to worsening of disease activity. Four patients achieved complete remission (CR) on 1 to 20 mg/day of CS, three achieved CR at Week 13 (25%), and one achieved CR at Week 21.

Principia Biopharma: "A Study of PRN1008 in Adult Patients With Pemphigus Vulgaris",ClinicalTrials.gov, 10 March 2016 (2016-03-10), XP055764616 and KENTARO IZUMI ET AL: "Current Clinical Trials in Pemphigus and Pemphigoid", FRONTIERS IN IMMUNOLOGY, vol.10, 3 May 2019 (2019-05-03), page 978, XP055761713,DOI: 10.3389/fimmu.2019.00978 report on clinical trials of PRN1008 in the treatment of pemphigus.

PRN1008's efficacy effects are produced via three simultaneous mechanisms of action: anti-inflammatory effects; neutralization of pathogenic autoantibodies; and blockade of autoantibody production. PRN1008 inhibits inflammatory cellular activities in mast cells and neutrophils (Langrish C et al., 2019). It also neutralizes autoimmune responses by blocking signals from antibody's Fc region and reduces autoantibody generation by blocking B-cell activation and maturation (Langrish C et al., 2019; Langrish CL et al., 2017). These effects are produced without directly impacting T cells or causing B cell depletion. PRN1008 improved disease symptoms and outcomes in rats with collagen-induced arthritis (Hill R et al., 2015) and in canines with naturally occurring PF (Murrell DF, 2019), suggesting that PRN1008 inhibits inflammation and reverses tissue damage (Langrish CL et al., 2017).

Results of Phase 2 studies with PRN1008 for the treatment of PV and PF are discussed herein.

### BRIEF DESCRIPTION OF THE DRAWINGS

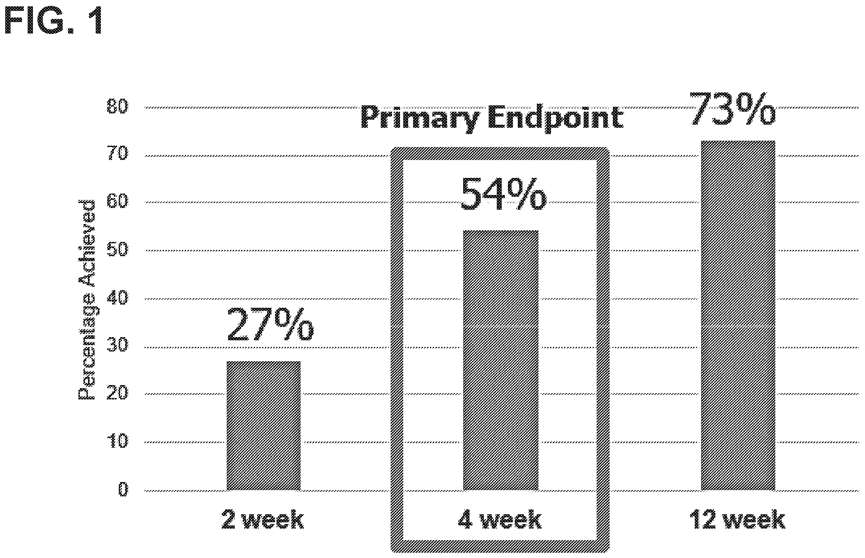

**FIG. 1** depicts the percentage of patients (n = 26) enrolled in a Phase 2 clinical trial (Part A) in which patients were administered 400 mg of PRN1008 BID with possible dose adjustment up to 600 mg of PRN1008 BID at investigator discretion for a 12-week treatment period who achieved control of disease activity (CDA) at 2 weeks (27%), 4 weeks (54%), and 12 weeks (73%). One patient enrolled in Part A who dropped out on day 10 due to a treatment-unrelated adverse event (AE) was excluded from the analysis.

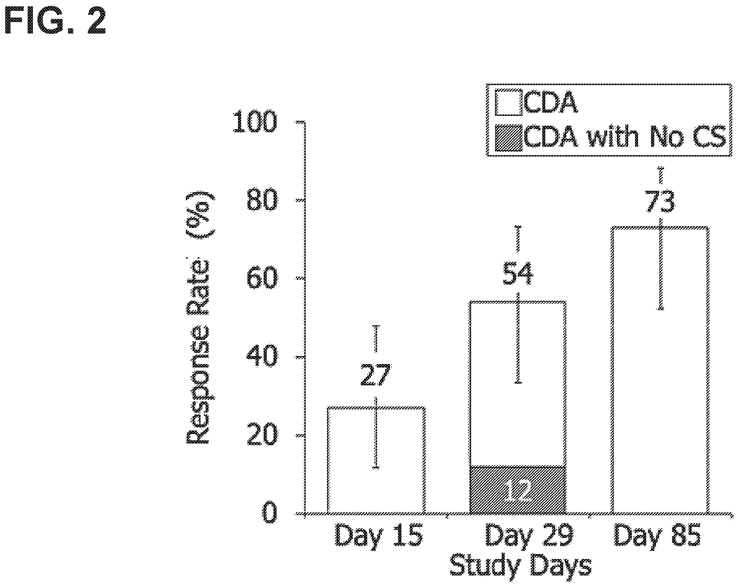

**FIG. 2** depicts the control of disease activity response rate (%) on study day 15 (27%), study day 29 (54%), or study day 85 (73%) for patients enrolled in a Phase 2 clinical trial (Part A) in which patients were administered 400 mg of PRN1008 BID with possible dose adjustment up to 600 mg of PRN1008 BID at investigator discretion for a 12-week treatment period. Error bars indicate 95% confidence interval (CI). At study days 15 and 29, n = 26. At study day 85, n = 24.

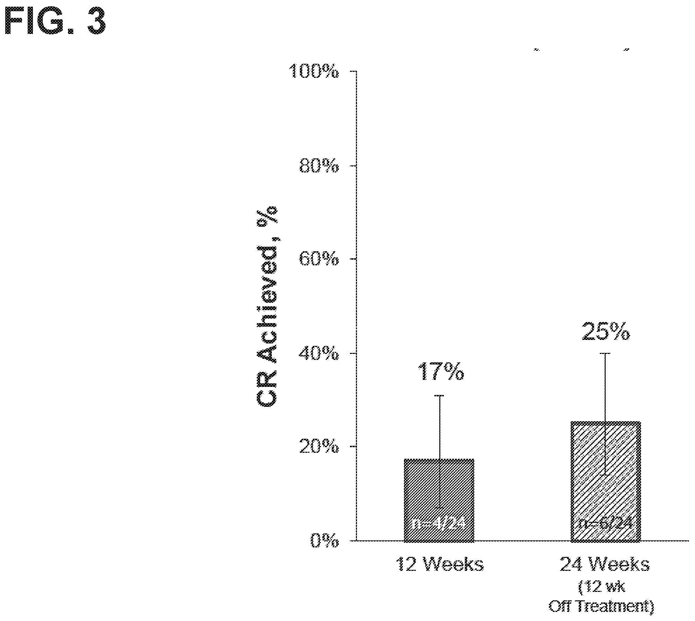

**FIG. 3** depicts the percentage of patients (n = 24) enrolled in a Phase 2 clinical trial (Part A) in which patients were administered 400 mg of PRN1008 BID with possible dose adjustment up to 600 mg of PRN1008 BID at investigator discretion for a 12-week treatment period who achieved complete remission (CR) at 12 weeks (17%) and 24 weeks (12 weeks on treatment, 12 weeks off treatment) (25%). Three patients enrolled in Part A were excluded from the analysis due to a treatment-unrelated adverse event after 10, 43, and 44 days.

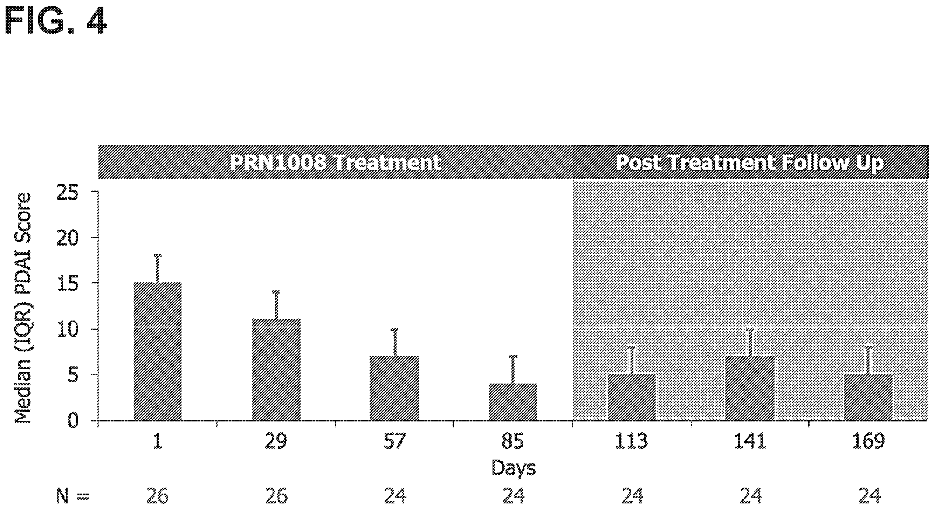

**FIG. 4** depicts PDAI scores (median (interquartile range)) over time for patients enrolled in a Phase 2 clinical trial (Part A) in which patients were administered 400 mg of PRN1008 BID with possible dose adjustment up to 600 mg of PRN1008 BID at investigator discretion for a 12-week treatment period.

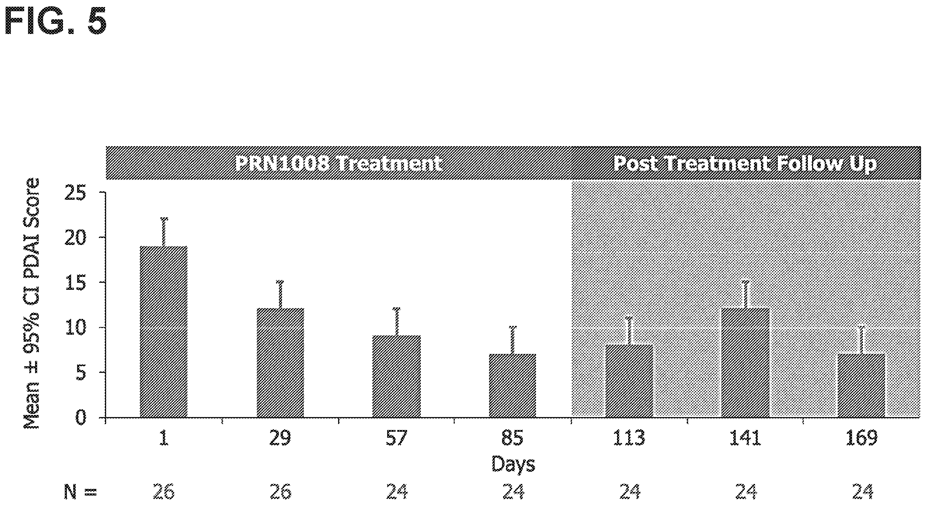

**FIG. 5** depicts PDAI scores (mean ± 95% CI) over time for patients enrolled in a Phase 2 clinical trial (Part A) in which patients were administered 400 mg of PRN1008 BID with possible dose adjustment up to 600 mg of PRN1008 BID at investigator discretion for a 12-week treatment period.

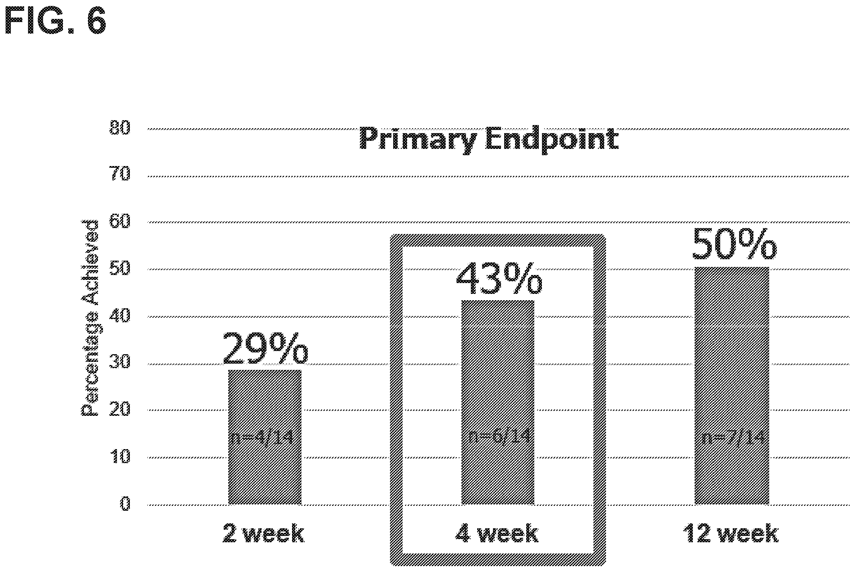

**FIG. 6** depicts the percentage of patients (n = 14) enrolled in a Phase 2 clinical trial (Part B) in which patients were administered 400 mg of PRN1008 QD for a 24-week treatment period who achieved control of disease activity (CDA) at 2 weeks (29%), 4 weeks (43%), and 12 weeks (50%). One patient that rolled from Part A to Part B and started on 400 mg of PRN1008 BID was excluded from the analysis.

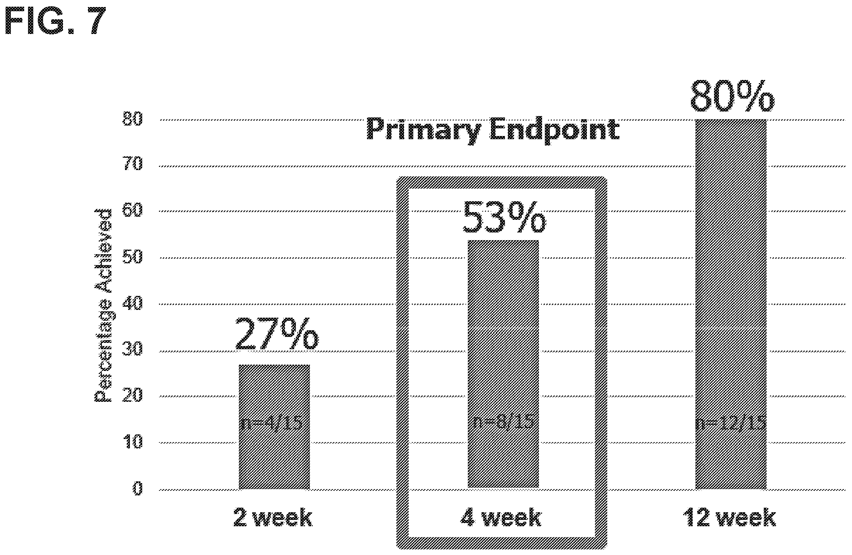

**FIG. 7** depicts the percentage of patients (n = 15) enrolled in a Phase 2 clinical trial (Part B) in which patients were administered 400 mg of PRN1008 QD with possible dose adjustment up to 600 mg of PRN1008 BID at investigator discretion for a 24-week treatment period who achieved control of disease activity (CDA) at 2 weeks (27%), 4 weeks (53%), and 12 weeks (80%). The patient that rolled from Part A to Part B and started on 400 mg of PRN1008 BID was included in the analysis.

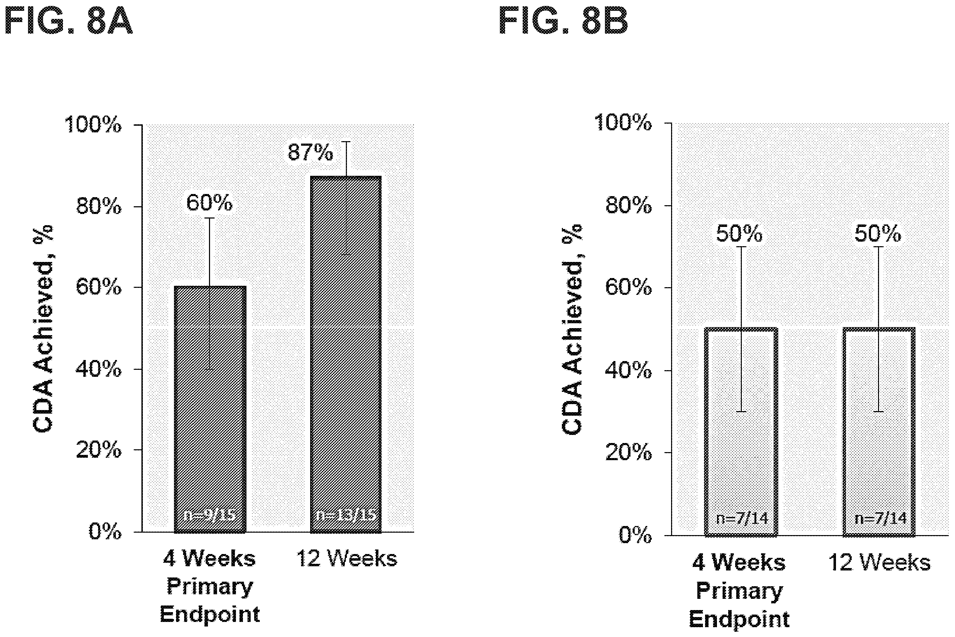

**FIG. 8A** depicts the percentage of patients (n = 15) enrolled in a Phase 2 clinical trial (Part B) in which patients were administered 400 mg of PRN1008 QD with possible dose adjustment up to 600 mg of PRN1008 BID at investigator discretion for a 24-week treatment period who achieved control of disease activity (CDA) at 4 weeks (60%) and 12 weeks (87%). The patient that rolled from Part A to Part B and started on 400 mg of PRN1008 BID was included in the analysis. Error bars represent 80% CI calculated by the Clopper-Pearson method.
**FIG. 8B** depicts the percentage of patients (n = 14) enrolled in a Phase 2 clinical trial (Part B) in which patients were administered 400 mg of PRN1008 QD for a 24-week treatment period who achieved control of disease activity (CDA) at 4 weeks (50%) and 12 weeks (50%). The patient that rolled from Part A to Part B and started on 400 mg of PRN1008 BID was excluded from the analysis. Error bars represent 80% CI calculated by the Clopper-Pearson method.

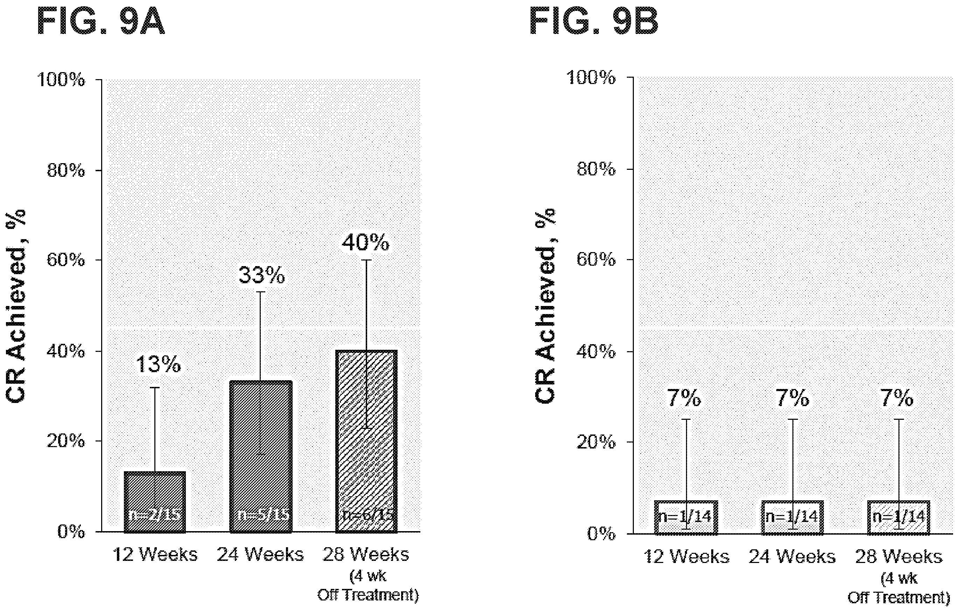

**FIG. 9A** depicts the percentage of patients (n = 14) enrolled in a Phase 2 clinical trial (Part B) in which patients were administered 400 mg of PRN1008 QD with possible dose adjustment up to 600 mg of PRN1008 BID at investigator discretion for a 24-week treatment period who achieved complete remission (CR) at 12 weeks (13%), 24 weeks (33%), and 28 weeks (24 weeks on treatment, 4 weeks off treatment) (40%). The patient that rolled from Part A to Part B and started on 400 mg of PRN1008 BID was included in the analysis.
**FIG. 9B** depicts the percentage of patients (n = 14) enrolled in a Phase 2 clinical trial (Part B) in which patients were administered 400 mg of PRN1008 QD for a 24-week treatment period who achieved complete remission (CR) at 12 weeks (7%), 24 weeks (7%), and 28 weeks (24 weeks on treatment, 4 weeks off treatment) (7%). The patient that rolled from Part A to Part B and started on 400 mg of PRN1008 BID was excluded from the analysis.

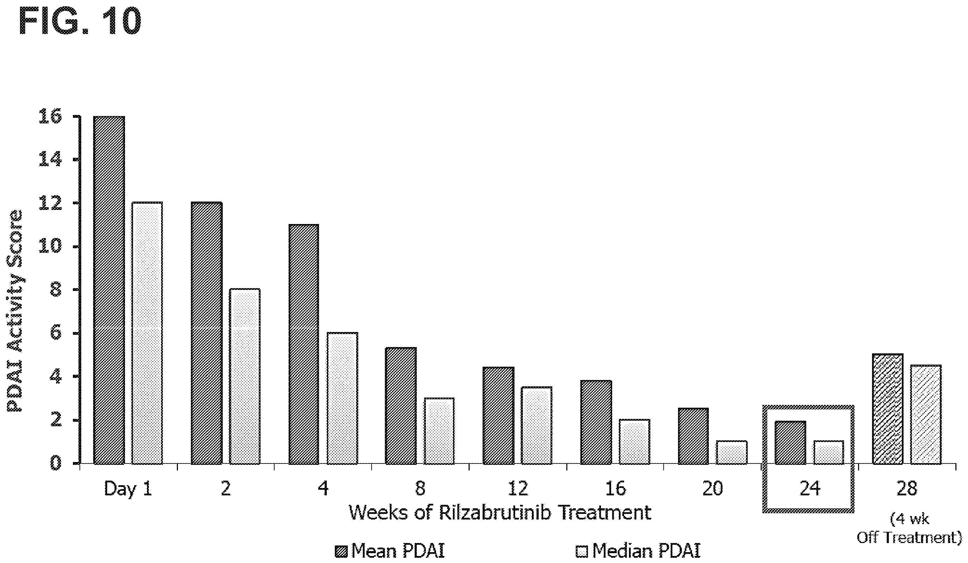

**FIG. 10** depicts mean and median PDAI scores for patients (n = 16 through 8 weeks, n = 15 thereafter) enrolled in a Phase 2 clinical trial (Part B) in which patients were administered 400 mg of PRN1008 QD with possible dose adjustment up to 600 mg of PRN1008 BID at investigator discretion for a 24-week treatment period. One patient dropped out of the study after 8 weeks due to worsening pemphigus and was not included in PDAI score calculations are 8 weeks.

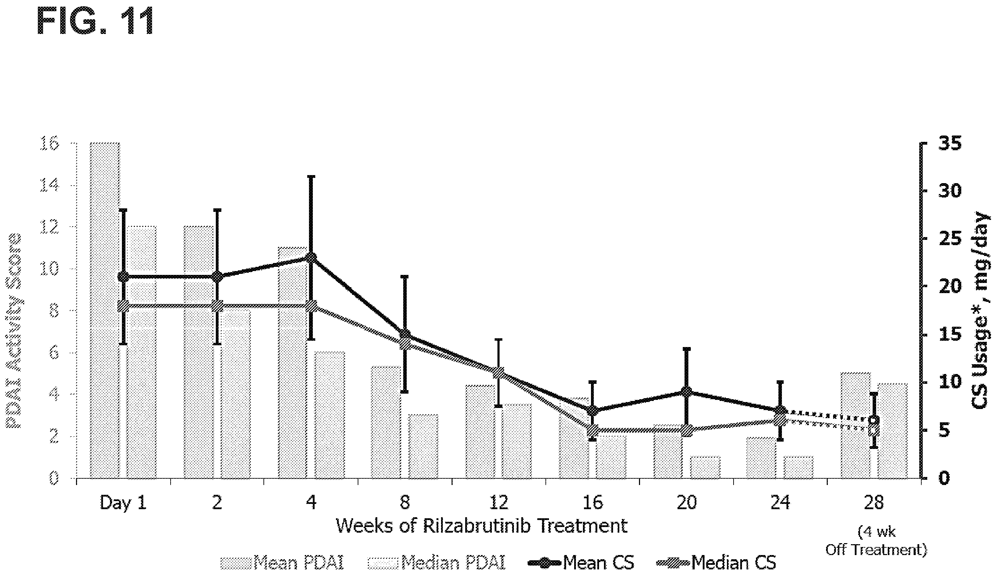

**FIG. 11** depicts mean and median PDAI scores and mean and median corticosteroid (CS) usage for patients (n = 16 through 8 weeks, n = 15 thereafter) enrolled in a Phase 2 clinical trial (Part B) in which patients were administered 400 mg of PRN1008 QD with possible dose adjustment up to 600 mg of PRN1008 BID at investigator discretion for a 24-week treatment period. One patient dropped out of the study after 8 weeks due to worsening pemphigus and was not included in PDAI score/CS usage calculations are 8 weeks.

### Definitions:

Unless otherwise stated, the following terms used in the specification and claims are defined for the purposes of this Application and have the following meanings. All undefined technical and scientific terms used in this Application have the meaning as commonly understood by one of ordinary skill in the art to which this disclosure belongs.

As used herein, "a" or "an" entity refers to one or more of that entity; for example, a compound refers to one or more compounds or at least one compound unless stated otherwise. As such, the terms "a" (or "an"), "one or more," and "at least one" can be used interchangeably herein.

As used herein, the term "about" means approximately, in the region of, roughly, or around. When the term "about" is used in conjunction with a numerical range, it modifies that range by extending the boundaries above and below the numerical values set forth. In general, the term "about" is used herein to modify a numerical value above and below the stated value by a variance of 5%. With regard to specific values, it should be understood that specific values described herein for subject populations (e.g., the subject of the described clinical trial) represent median, mean, or statistical numbers, unless otherwise provided. Accordingly, aspects of the present disclosure requiring a particular value in a subject are supported herein by population data in which the relevant value is assessed to be a meaningful delimitation on the subject population.

As used herein, the term "active pharmaceutical ingredient" or "therapeutic agent" ("API") refers to a biologically active compound.

As used herein, the terms "administer," "administering," or "administration" herein refer to providing, giving, dosing, and/or prescribing by either a health practitioner or an authorized agent and/or putting into, taking or consuming by the patient or person himself or herself. For example, "administration" of an API to a patient refers to any route (e.g., oral delivery) of introducing or delivering the API to the patient. Administration includes self administration and administration by another.

As used herein, the term "characterized by relapsing pemphigus," in reference to a human patient, refers to a human patient with relapsing pemphigus, such as, e.g., relapsing pemphigus vulgaris or relapsing pemphigus foliaceus. As a non-limiting example, "characterized by relapsing pemphigus prior to the administration of PRN1008," in reference to a human patient, refers to a human patient suffering from relapsing pemphigus before beginning a dosing regimen comprising administering PRN1008.

As used herein, the term "characterized by a maintenance dose of less than or equal to 0.5 mg/kg/day (≤ 0.5 mg/kg/day) of a corticosteroid," in reference to a human patient, refers to a human patient administered a maintenance dose of less than or equal to 0.5 mg/kg/day (≤ 0.5 mg/kg/day) of a corticosteroid, including patients not previously administered corticosteroids (0 mg/kg/day). As a non-limiting example, "characterized by a maintenance dose of less than or equal to 0.5 mg/kg/day (≤ 0.5 mg/kg/day) of a corticosteroid prior to the administration of PRN1008," in reference to a human patient, refers to a human patient not on corticosteroids or a patient who was administered a maintenance dose of less than or equal to 0.5 mg/kg/day (≤ 0.5 mg/kg/day) of a corticosteroid before beginning a dosing regimen comprising administering PRN1008 and optionally administering a corticosteroid, which may be the same or different from the corticosteroid used as a maintenance therapy prior to the administration of PRN1008 and may be administered at a dose or on a dosing schedule that is the same or different from that used for maintenance therapy prior to the administration of PRN1008.

As used herein, unless otherwise specified, the term "complete remission" (CR) is defined as the absence of new and established lesions and is intended to mean "no disease activity."

As used herein, unless otherwise specified, the term "control of disease activity" (CDA) (disease control) is defined as the visit at which new pemphigus lesions cease to form and established pemphigus lesions begin to heal. This is also considered the beginning of the consolidation phase. The expected interval of time to reach the control of disease activity is on the order of weeks, although it may be shorter.

As used herein, unless otherwise specified, the term "end of consolidation phase" (ECP) is defined as the visit at which no new lesions have developed for a minimum of 2 weeks and the majority (e.g., at least 60%, at least 70%, at least 80%) of established lesions have healed. Therefore, in order to achieve ECP, CDA must be confirmed at a visit ≥ 2 weeks later and 80% of the lesions seen previously must have healed.

As used herein, the term "following the administration of [X]," when modifying a period of time, refers to a period of time beginning after the administration of [X] concludes. As a non-limiting example, "administering to the human patient a second dose of 400 mg of PRN1008 twice a day (BID) for 14 days following the administration of the first dose" refers to beginning administration of the second dose after administration of the first dose concludes and administering the second dose for 14 days, i.e., if, e.g., the first dose was administered once daily on days 1 to 14, then the second dose will be administered twice daily on days 15 to 28, for a total treatment period of 28 days.

As used herein, the term "in combination with," when referring to two or more compounds, agents, or additional active pharmaceutical ingredients, means the administration of two or more compounds, agents, or active pharmaceutical ingredients to the patient prior to, concurrent with, or subsequent to each other during a treatment period. Unless specified otherwise, the two or more compounds, agents, or active pharmaceutical ingredients may be administered on different schedules during the treatment period, such as, e.g., with one or more compounds, agents, or active pharmaceutical ingredients being administered once a day and one or more other compounds, agents, or active pharmaceutical ingredients being administered twice a day.

As used herein, an amount expressed in terms of "mg of [X]" refers to the total amount in milligrams of [X], i.e., the free base. In some embodiments, PRN1008 may be administered as a pharmaceutically acceptable salt of PRN1008, in which case an amount expressed in terms of "mg of PRN1008" refers to the total amount in milligrams of PRN1008, i.e., the free base, plus the equivalent amount of one or more pharmaceutically acceptable salts of PRN1008 based on the weight of free base therein. For example, "400 mg of at least one compound chosen from PRN1008 and pharmaceutically acceptable salts thereof" includes 400 mg of PRN1008 and a concentration of one or more pharmaceutically acceptable salts of PRN1008 equivalent to 400 mg of PRN1008.

As used herein, the term "pemphigus lesion" refers to a lesion associated with or caused by pemphigus, such as, e.g., a lesion associated with or caused by pemphigus vulgaris or a lesion associated with or caused by pemphigus foliaceus.

As used herein, the term "pharmaceutically acceptable salt" refers to a salt form of an active pharmaceutical agent, wherein the salt is nontoxic. Pharmaceutically acceptable salts are well known in the art and include those derived from suitable inorganic and organic acids. For example, S. M. Berge, et al. describe pharmaceutically acceptable salts in detail in J. Pharmaceutical Sciences, 1977, 66, 1-19.

As used herein, the term "PRN1008" refers to a compound having the structure: 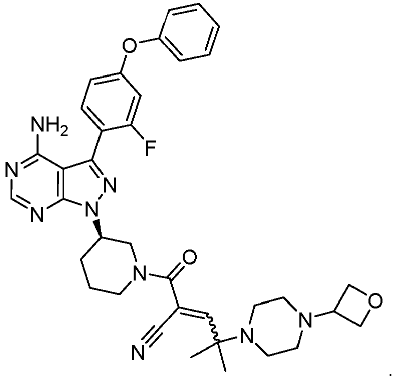 A dose of PRN1008 may contain the corresponding (S) enantiomer as an impurity in less than about 1% by weight or no more than about 5% by weight. Similarly, a dose of the (E) isomer of PRN1008 may contain the corresponding (Z) isomer as an impurity in less than about 1% by weight; a dose of the (Z) isomer of PRN1008 may contain the corresponding (E) isomer as an impurity in less than about 1% by weight. When PRN1008 is denoted as a mixture of (E) and (Z) isomers of (R)-2-[3-[4-amino-3-(2-fluoro-4-phenoxyphenyl)pyrazolo[3,4-d]pyrimidin-1-yl]piperidine-1-carbonyl]-4-methyl-4-[4-(oxetan-3-yl)piperazin-1-yl]pent-2-enenitrile, it means that the amount of (E) or (Z) isomer in the mixture is greater than about 1% by weight. In some embodiments, the molar ratio of (E) to (Z) isomer is 9: 1. PRN1008 or a pharmaceutically acceptable salt thereof may also be referred to herein as a "drug," "active agent," "a therapeutically active agent," or "API."

As used herein, the term "relapse" is defined by the appearance of 3 or more new lesions within a month that do not heal spontaneously within 1 week, or by the extension of established lesions, in a patient who has achieved disease control.

As used herein, the term "treat," "treating," or "treatment," when used in connection with a disorder or condition, includes any effect, e.g., lessening, reducing, modulating, ameliorating, or eliminating, that results in the improvement of the disorder or condition. Improvements in or lessening the severity of any symptom of the disorder or condition can be readily assessed according to standard methods and techniques known in the art.

The present invention relates to a compound which is (R)-2-[3-[4-amino-3-(2-fluoro-4-phenoxy-phenyl)pyrazolo[3,4-d]pyrimidin-1-yl]piperidine-1-carbonyl]-4-methyl-4-[4-(oxetan-3-yl)piperazin-1-yl]pent-2-enenitrile (PRN1008) or a pharmaceutically acceptable salt thereof, for treating pemphigus, wherein said compound is administered to a human patient twice a day (BID) at a dose of 400 mg, for at least 14 days, and wherein the human patient is characterized by relapsing pemphigus prior to the administration of said compound.

In some embodiments, the human patient is administered a dose of 400 mg of PRN1008 BID for 14 to 84 days. In some embodiments, the human patient is administered a dose of 400 mg of PRN1008 BID for 14 days. In some embodiments, the human patient is administered a dose of 400 mg of PRN1008 BID for 28 days. In some embodiments, the human patient is administered a dose of 400 mg of PRN1008 BID for 84 days.

In some embodiments, said compound is for use in methods comprising:
administering to the human patient a first dose of 400 mg of PRN1008 twice a day (BID) for at least 14 days; and
administering to the human patient a second dose of 500 mg of PRN1008 twice a day (BID) following the administration of the first dose.

In some embodiments, the human patient is administered a first dose of 400 mg of PRN1008 BID for 14 to 28 days. In some embodiments, the human patient is administered a first dose of 400 mg of PRN1008 BID for more than 28 days. In some embodiments, the human patient is administered a first dose of 400 mg of PRN1008 BID for 33 days.

In some embodiments, PRN1008 is administered to the human patient for at most 84 days.

In some embodiments, said compound is for use in method comprising:
administering to the human patient a first dose of 400 mg of PRN1008 twice a day (BID) for at least 14 days; and
administering to the human patient a second dose of 600 mg of PRN1008 twice a day (BID) following the administration of the first dose.

In some embodiments, the human patient is administered a first dose of 400 mg of PRN1008 BID for 14 to 28 days. In some embodiments, the human patient is administered a first dose of 400 mg of PRN1008 BID for more than 28 days. In some embodiments, the human patient is administered a first dose of 400 mg of PRN1008 BID for 22 days. In some embodiments, the human patient is administered a first dose of 400 mg of PRN1008 BID for 56 days.

In some embodiments, PRN1008 is administered to the human patient for at most 84 days.

In some embodiments, said compound is for use in methods comprising:
administering to the human patient a first dose of 400 mg of PRN1008 twice a day (BID) for at least 14 days;
administering to the human patient a second dose of 500 mg of PRN1008 twice a day (BID) for at least 14 days following the administration of the first dose; and
administering to the human patient a third dose of 600 mg of PRN1008 twice a day (BID) following the administration of the second dose.

In some embodiments, the human patient is administered a first dose of 400 mg of PRN1008 BID for 14 to 28 days. In some embodiments, the human patient is administered a first dose of 400 mg of PRN1008 BID for more than 28 days.

In some embodiments, the human patient is administered a second dose of 500 mg of PRN1008 BID for 14 to 28 days following the administration of the first dose.

In some embodiments, PRN1008 is administered to the human patient for at most 84 days.

In some embodiments, said compound is for use in methods comprising administering PRN1008 in combination with a first corticosteroid at a dose of less than or equal to 0.5 mg/kg/day (≤ 0.5 mg/kg/day).

In some embodiments, the first corticosteroid is chosen from prednisone, prednisolone, and methylprednisolone.

In some embodiments, the human patient is characterized by relapsing pemphigus vulgaris prior to the administration of PRN1008.

In some embodiments, the human patient is characterized by relapsing pemphigus foliaceus prior to the administration of PRN1008.

In some embodiments, said compound is for use in a method comprising treating pemphigus vulgaris.

In some embodiments, said compound is for use in a method comprising treating pemphigus foliaceus.

In some embodiments, the human patient is characterized by a pemphigus disease activity index (PDAI) skin score from 8 points to 60 points prior to the administration of PRN1008. In some embodiments, the human patient is characterized by a pemphigus disease activity index (PDAI) skin score from 8 points to 45 points prior to the administration of PRN1008.

In some embodiments, the human patient is characterized by a maintenance dose of less than or equal to 0.5 mg/kg/day (≤ 0.5 mg/kg/day) of a second corticosteroid prior to the administration of PRN1008.

In some embodiments, the second corticosteroid is chosen from prednisone, prednisolone, and methylprednisolone.

In some embodiments, the first corticosteroid is the same as the second corticosteroid. In some embodiments, the first corticosteroid is not the same as the second corticosteroid.

In some embodiments, PRN1008 comprises the (E) isomer of (R)-2-[3-[4-amino-3-(2-fluoro-4-phenoxy-phenyl)pyrazolo[3,4-d]pyrimidin-1-yl]piperidine-1-carbonyl]-4-methyl-4-[4-(oxetan-3-yl)piperazin-1-yl]pent-2-enenitrile. In some embodiments, PRN1008 comprises the (Z) isomer of (R)-2-[3-[4-amino-3-(2-fluoro-4-phenoxyphenyl)pyrazolo[3,4-d]pyrimidin-1-yl]piperidine-1-carbonyl]-4-methyl-4-[4-(oxetan-3-yl)piperazin-1-yl]pent-2-enenitrile. In some embodiments, PRN1008 comprises a mixture of (E) and (Z) isomers of (R)-2-[3-[4-amino-3-(2-fluoro-4-phenoxy-phenyl)pyrazolo[3,4-d]pyrimidin-1-yl]piperidine-1-carbonyl]-4-methyl-4-[4-(oxetan-3-yl)piperazin-1-yl]pent-2-enenitrile.

In some embodiments, said compound is for use in methods comprising reducing a pemphigus disease activity index (PDAI) skin score. In some embodiments, said compound is for use in methods comprising reducing a pemphigus disease activity index (PDAI) skin score by at least 20%. In some embodiments, said compound is for use in methods comprising reducing a pemphigus disease activity index (PDAI) skin score by at least 25%. In some embodiments, said compound is for use in methods comprising reducing a pemphigus disease activity index (PDAI) skin score by at least 30%. In some embodiments, said compound is for use in methods comprising reducing a pemphigus disease activity index (PDAI) skin score by at least 35%. In some embodiments, said compound is for use in methods comprising reducing a pemphigus disease activity index (PDAI) skin score by at least 40%. In some embodiments, said compound is for use in methods comprising reducing a pemphigus disease activity index (PDAI) skin score by at least 45%. In some embodiments, said compound is for use in methods comprising reducing a pemphigus disease activity index (PDAI) skin score by at least 50%. In some embodiments, said compound is for use in methods comprising reducing a pemphigus disease activity index (PDAI) skin score by at least 55%. In some embodiments, said compound is for use in methods comprising reducing a pemphigus disease activity index (PDAI) skin score by at least 60%. In some embodiments, said compound is for use in methods comprising reducing a pemphigus disease activity index (PDAI) skin score by at least 65%. In some embodiments, said compound is for use in methods comprising reducing a pemphigus disease activity index (PDAI) skin score by at least 70%. In some embodiments, said compound is for use in methods comprising reducing a pemphigus disease activity index (PDAI) skin score by at least 75%. In some embodiments, said compound is for use in methods comprising reducing a pemphigus disease activity index (PDAI) skin score by at least 80%. In some embodiments, said compound is for use in methods comprising reducing a pemphigus disease activity index (PDAI) skin score by at least 85%. In some embodiments, said compound is for use in methods comprising reducing a pemphigus disease activity index (PDAI) skin score by at least 90%.

In some embodiments, said compound is for use in methods comprising reducing a pemphigus disease activity index (PDAI) skin score after 14 days of PRN1008 administration. In some embodiments, said compound is for use in methods comprising reducing a pemphigus disease activity index (PDAI) skin score after 28 days of PRN1008 administration. In some embodiments, said compound is for use in methods comprising reducing a pemphigus disease activity index (PDAI) skin score after 56 days of PRN1008 administration. In some embodiments, said compound is for use in methods comprising reducing a pemphigus disease activity index (PDAI) skin score after 84 days of PRN1008 administration. In some embodiments, said compound is for use in methods comprising reducing a pemphigus disease activity index (PDAI) skin score after 112 days of PRN1008 administration. In some embodiments, said compound is for use in methods comprising reducing a pemphigus disease activity index (PDAI) skin score after 140 days of PRN1008 administration. In some embodiments, said compound is for use in methods comprising reducing a pemphigus disease activity index (PDAI) skin score after 168 days of PRN1008 administration.

In some embodiments, said compound is for use in methods comprising reducing a pemphigus disease activity index (PDAI) skin score by at least 20% after 14 days of PRN1008 administration. In some embodiments, said compound is for use in methods comprising reducing a pemphigus disease activity index (PDAI) skin score by at least 20% after 28 days of PRN1008 administration. In some embodiments, said compound is for use in methods comprising reducing a pemphigus disease activity index (PDAI) skin score by at least 50% after 56 days of PRN1008 administration. In some embodiments, said compound is for use in methods comprising reducing a pemphigus disease activity index (PDAI) skin score by at least 50% after 84 days of PRN1008 administration. In some embodiments, said compound is for use in methods comprising reducing a pemphigus disease activity index (PDAI) skin score by at least 70% after 168 days of PRN1008 administration. In some embodiments, said compound is for use in methods comprising reducing a pemphigus disease activity index (PDAI) skin score by at least 75% after 168 days of PRN1008 administration.

In some embodiments, the human patient is characterized by a pemphigus disease activity index (PDAI) skin score of 0 or 1 following the administration of PRN1008. In some embodiments, the human patient is characterized by a pemphigus disease activity index (PDAI) skin score of 0 following the administration of PRN1008. In some embodiments, the human patient is characterized by a pemphigus disease activity index (PDAI) skin score of 1 following the administration of PRN1008.

In some embodiments, the human patient is characterized by a pemphigus disease activity index (PDAI) skin score of 0 or 1 after 168 days of PRN1008 administration. In some embodiments, the human patient is characterized by a pemphigus disease activity index (PDAI) skin score of 0 after 168 days of PRN1008 administration. In some embodiments, the human patient is characterized by a pemphigus disease activity index (PDAI) skin score of 1 after 168 days of PRN1008 administration.

In some embodiments, said compound is for use in methods comprising reducing average daily corticosteroid usage by the human patient. In some embodiments, said compound is for use in methods comprising reducing average daily corticosteroid usage by the human patient by at least 20%. In some embodiments, said compound is for use in methods comprising reducing average daily corticosteroid usage by the human patient. In some embodiments, said compound is for use in methods comprising reducing average daily corticosteroid usage by the human patient by at least 20%. In some embodiments, said compound is for use in methods comprising reducing average daily corticosteroid usage by the human patient by at least 25%. In some embodiments, said compound is for use in methods comprising reducing average daily corticosteroid usage by the human patient by at least 30%. In some embodiments, said compound is for use in methods comprising reducing average daily corticosteroid usage by the human patient by at least 35%. In some embodiments, said compound is for use in methods comprising reducing average daily corticosteroid usage by the human patient by at least 40%. In some embodiments, said compound is for use in methods comprising reducing average daily corticosteroid usage by the human patient by at least 45%. In some embodiments, said compound is for use in methods comprising reducing average daily corticosteroid usage by the human patient by at least 50%. In some embodiments, said compound is for use in methods comprising reducing average daily corticosteroid usage by the human patient by at least 55%. In some embodiments, said compound is for use in methods comprising reducing average daily corticosteroid usage by the human patient by at least 60%. In some embodiments, said compound is for use in methods comprising reducing average daily corticosteroid usage by the human patient by at least 65%.

In some embodiments, said compound is for use in methods comprising reducing average daily corticosteroid usage by the human patient by at least 20% after 56 days of PRN1008 administration. In some embodiments, said compound is for use in methods comprising reducing average daily corticosteroid usage by the human patient by at least 30% after 84 days of PRN1008 administration. In some embodiments, said compound is for use in methods comprising reducing average daily corticosteroid usage by the human patient by at least 50% after 112 days of PRN1008 administration.

In some embodiments, said compound is for use in methods comprising achieving complete remission. In some embodiments, said compound is for use in methods comprising achieving complete remission after 84 days of PRN1008 administration. In some embodiments, said compound is for use in methods comprising achieving complete remission after 168 days of PRN1008 administration.

In some embodiments, said compound is for use in methods comprising achieving a control of disease activity of pemphigus. In some embodiments, said compound is for use in methods comprising achieving control of disease activity defined as a visit for a medical checkup at which new pemphigus lesions cease to form and established pemphigus lesions begin to heal. In some embodiments, said compound is for use in methods comprising achieving control of disease activity after 84 days of PRN1008 administration. In some embodiments, said compound is for use in methods comprising achieving control of disease activity after 168 days of PRN1008 administration.

In some embodiments, said compound is for use in methods comprising healing established pemphigus lesions. In some embodiments, said compound is for use in methods comprising healing at least 50% of established pemphigus lesions. In some embodiments, said compound is for use in methods comprising healing at least 60% of established pemphigus lesions. In some embodiments, said compound is for use in methods comprising healing at least 70% of established pemphigus lesions. In some embodiments, said compound is for use in methods comprising healing at least 80% of established pemphigus lesions. In some embodiments, said compound is for use in methods comprising healing at least 90% of established pemphigus lesions.

In some embodiments, said compound is for use in methods comprising healing at least 50% of established pemphigus lesions after 84 days of PRN1008 administration. In some embodiments, said compound is for use in methods comprising healing at least 60% of established pemphigus lesions after 84 days of PRN1008 administration. In some embodiments, said compound is for use in methods comprising healing at least 70% of established pemphigus lesions after 84 days of PRN1008 administration. In some embodiments, said compound is for use in methods comprising healing at least 80% of established pemphigus lesions after 84 days of PRN1008 administration. In some embodiments, said compound is for use in methods comprising healing at least 90% of established pemphigus lesions after 84 days of PRN1008 administration.

In some embodiments, said compound is for use in methods comprising healing at least 50% of established pemphigus lesions after 168 days of PRN1008 administration. In some embodiments, said compound is for use in methods comprising healing at least 60% of established pemphigus lesions after 168 days of PRN1008 administration. In some embodiments, said compound is for use in methods comprising healing at least 70% of established pemphigus lesions after 168 days of PRN1008 administration. In some embodiments, said compound is for use in methods comprising healing at least 80% of established pemphigus lesions after 168 days of PRN1008 administration. In some embodiments, said compound is for use in methods comprising healing at least 90% of established pemphigus lesions after 168 days of PRN1008 administration.

In some embodiments, said compound is for use in methods comprising preventing new pemphigus lesion formation. In some embodiments, said compound is for use in methods comprising preventing new pemphigus lesion formation after 84 days of PRN1008 administration. In some embodiments, said compound is for use in methods comprising preventing new pemphigus lesion formation after 168 days of PRN1008 administration.

In some embodiments, said compound is for use in methods comprising achieving an end of consolidation phase of pemphigus. In some embodiments, said compound is for use in methods comprising achieving end of consolidation phase defined as a medical visit at which no new pemphigus lesion have developed for a minimum of 2 weeks and a majority of established pemphigus lesions have healed. In some embodiments, more than 50% of established pemphigus lesions have healed. In some embodiments, more than 60% of established pemphigus lesions have healed. In some embodiments, more than 70% of established pemphigus lesions have healed. In some embodiments, more than 80% of established pemphigus lesions have healed. In some embodiments, more than 90% of established pemphigus lesions have healed.

In some embodiments, said compound is for use in methods comprising achieving end of consolidation phase defined as a medical visit at which no new pemphigus lesion have developed for a minimum of 2 weeks and a majority of established pemphigus lesions have healed after 168 days of PRN1008 administration. In some embodiments, more than 50% of established pemphigus lesions have healed. In some embodiments, more than 60% of established pemphigus lesions have healed. In some embodiments, more than 70% of established pemphigus lesions have healed. In some embodiments, more than 80% of established pemphigus lesions have healed. In some embodiments, more than 90% of established pemphigus lesions have healed.

Some embodiments of the present disclosure relate to compound (R)-2-[3-[4-amino-3-(2-fluoro-4-phenoxy-phenyl)pyrazolo[3,4-d]pyrimidin-1-yl]piperidine-1-carbonyl]-4-methyl-4-[4-(oxetan-3-yl)piperazin-1-yl]pent-2-enenitrile (PRN1008) for use in methods for achieving a primary endpoint in 43% to 63% of a human patient population undergoing treatment for pemphigus comprising:
administering to each member of the patient population a dose of 400 mg of said compound once a day (QD) for 14 days, optionally in combination with a first corticosteroid at a dose of less than or equal to 0.5 mg/kg/day (≤ 0.5 mg/kg/day), followed by a dose of 400 mg of PRN1008 twice a day (BID) for 14 days in combination with the first corticosteroid at a dose of less than or equal to 0.5 mg/kg/day (≤ 0.5 mg/kg/day), wherein each member of the human patient population was characterized by:
relapsing pemphigus;
a pemphigus disease activity index (PDAI) skin score of 8-60 points; and
a maintenance dose of less than or equal to 0.5 mg/kg/day (≤ 0.5 mg/kg/day) of a second corticosteroid,
prior to the administration of PRN1008,
wherein the primary endpoint comprises control of disease activity (CDA) defined as a visit for a medical checkup at which new pemphigus lesions cease to form and established pemphigus lesions begin to heal.

In some embodiments, said compound is for use in methods comprising achieving the primary endpoint in 48% to 58% of the human patient population.

In some embodiments, said compound is for use in methods comprising achieving the primary endpoint in 53% of the human patient population.

In some embodiments, the human patient population is undergoing treatment for pemphigus vulgaris or pemphigus foliaceus.

In some embodiments, each member of the human patient population was characterized by relapsing pemphigus vulgaris or relapsing pemphigus foliaceus prior to the administration of PRN1008. In some embodiments, each member of the human patient population was characterized by relapsing pemphigus vulgaris prior to the administration of PRN1008.

In some embodiments, the first corticosteroid administered to a member of the human patient population and the second corticosteroid administered to the member of the human patient population are independently chosen from prednisone, prednisolone, and methylprednisolone. In some embodiments, the first corticosteroid administered to a member of the human patient population is the same as the second corticosteroid administered to the member of the human patient population. In some embodiments, the first corticosteroid administered to a member of the human patient population is not the same as the second corticosteroid administered to the member of the human patient population.

Also included is compound (R)-2-[3-[4-amino-3-(2-fluoro-4-phenoxyphenyl)pyrazolo[3,4-d]pyrimidin-1-yl]piperidine-1-carbonyl]-4-methyl-4-[4-(oxetan-3-yl)piperazin-1-yl]pent-2-enenitrile (PRN1008) for use in a method for achieving a primary endpoint in 53% of a human patient population undergoing treatment for pemphigus vulgaris (PV) or pemphigus or pemphigus foliaceus (PF) comprising administering to the patient population 400 mg of said compound once a day (QD) for 14 days, followed by escalating the dose of PRN1008 to 400 mg BID for 14 days, wherein throughout the administration of PRN1008 to the patient population, the patient population also administered a corticosteroid at a dose of less than or equal to 0.5 mg/kg/day (≤ 0.5 mg/kg/day); wherein prior to the 400 mg QD administration of PRN1008, the patient population has (a) relapsing PV; and (b) a pemphigus disease activity index (PDAI) skin score of 8-60 points, and is maintained on a low dose corticosteroid (LDCS) therapy that comprises administering a corticosteroid at a dose of ≤ 0.5 mg/kg/day; wherein the primary endpoint comprises control of disease activity (CDA) defined as a visit for medical checkup at which new lesions from PV or PF cease to form and established lesions from PV and PF begin to heal.

Some embodiments of the present disclosure relate to compound (R)-2-[3-[4-amino-3-(2-fluoro-4-phenoxy-phenyl)pyrazolo[3,4-d]pyrimidin-1-yl]piperidine-1-carbonyl]-4-methyl-4-[4-(oxetan-3-yl)piperazin-1-yl]pent-2-enenitrile (PRN1008) for use in methods for achieving a primary endpoint in 70% to 90% of a human patient population undergoing treatment for pemphigus comprising:
administering to each member of the human patient population a dose of 400 mg of said compound once a day (QD) for 14 days, optionally in combination with a first corticosteroid at a dose of less than or equal to 0.5 mg/kg/day (≤ 0.5 mg/kg/day), followed by a dose of 400 mg of PRN1008 twice a day (BID) for 14 days in combination with the first corticosteroid at a dose of less than or equal to 0.5 mg/kg/day (≤ 0.5 mg/kg/day), further followed by a dose of 600 mg of PRN1008 twice a day (BID) for 56 days in combination with the first corticosteroid at a dose of less than or equal to 0.5 mg/kg/day (≤ 0.5 mg/kg/day), wherein each member of the human patient population was characterized by:
relapsing pemphigus;
a pemphigus disease activity index (PDAI) skin score of 8-60 points; and
a maintenance dose of less than or equal to 0.5 mg/kg/day (≤ 0.5 mg/kg/day) of a second corticosteroid,
prior to the administration of PRN1008,
wherein the primary endpoint comprises control of disease activity (CDA) defined as a visit for a medical checkup at which new pemphigus lesions cease to form and established pemphigus lesions begin to heal.

In some embodiments, said compound is for use in methods comprising achieving the primary endpoint in 75% to 85% of the human patient population.

In some embodiments, said compound is for use in methods comprising achieving the primary endpoint in 80% of the human patient population.

In some embodiments, the human patient population is undergoing treatment for pemphigus vulgaris or pemphigus foliaceus.

In some embodiments, each member of the human patient population was characterized by relapsing pemphigus vulgaris or relapsing pemphigus foliaceus prior to the administration of PRN1008. In some embodiments, each member of the human patient population was characterized by relapsing pemphigus vulgaris prior to the administration of PRN1008.

In some embodiments, the first corticosteroid administered to a member of the human patient population and the second corticosteroid administered to the member of the human patient population are independently chosen from prednisone, prednisolone, and methylprednisolone. In some embodiments, the first corticosteroid administered to a member of the human patient population is the same as the second corticosteroid administered to the member of the human patient population. In some embodiments, the first corticosteroid administered to a member of the human patient population is not the same as the second corticosteroid administered to the member of the human patient population.

Also included is compound (R)-2-[3-[4-amino-3-(2-fluoro-4-phenoxyphenyl)pyrazolo[3,4-d]pyrimidin-1-yl]piperidine-1-carbonyl]-4-methyl-4-[4-(oxetan-3-yl)piperazin-1-yl]pent-2-enenitrile (PRN1008) for use in a method for achieving a primary endpoint in 80% of a human patient population undergoing treatment for pemphigus vulgaris (PV) or pemphigus or pemphigus foliaceus (PF) comprising administering to the patient population 400 mg of said compound once a day (QD) for 14 days, followed by escalating the dose of PRN1008 to 400 mg BID for 14 days, further followed by escalating the dose of PRN1008 to 600 mg BID for 56 days, wherein throughout the administration of PRN1008 to the patient population, the patient population is also administered a corticosteroid at a dose of less than or equal to 0.5 mg/kg/day (≤ 0.5 mg/kg/day); wherein prior to the 400 mg QD administration of PRN1008, the patient population has (a) relapsing PV; and (b) a pemphigus disease activity index (PDAI) skin score of 8-60 points, and is maintained on a low dose corticosteroid (LDCS) therapy that comprises administering a corticosteroid at a dose of ≤ 0.5 mg/kg/day; wherein the primary endpoint comprises control of disease activity (CDA) defined as a visit for medical checkup at which new lesions from PV or PF cease to form and established lesions from PV and PF begin to heal.

Some embodiments of the present disclosure relate to compound (R)-2-[3-[4-amino-3-(2-fluoro-4-phenoxy-phenyl)pyrazolo[3,4-d]pyrimidin-1-yl]piperidine-1-carbonyl]-4-methyl-4-[4-(oxetan-3-yl)piperazin-1-yl]pent-2-enenitrile (PRN1008) for use in methods for achieving complete remission of disease in 3% to 23% of a human patient population undergoing treatment for pemphigus comprising:
administering to each member of the human patient population a dose of 400 mg said compound once a day (QD) for 14 days, optionally in combination with a first corticosteroid at a dose of less than or equal to 0.5 mg/kg/day (≤ 0.5 mg/kg/day), followed by a dose of 400 mg of PRN1008 twice a day (BID) for 14 days in combination with the first corticosteroid at a dose of less than or equal to 0.5 mg/kg/day (≤ 0.5 mg/kg/day), further followed by a dose of 600 mg of PRN1008 twice a day (BID) for 56 days in combination with the first corticosteroid at a dose of less than or equal to 0.5 mg/kg/day (≤ 0.5 mg/kg/day), wherein each member of the human patient population was characterized by:
relapsing pemphigus;
a pemphigus disease activity index (PDAI) skin score of 8-60 points; and
a maintenance dose of less than or equal to 0.5 mg/kg/day (≤ 0.5 mg/kg/day) of a second corticosteroid,
prior to the administration of PRN1008,
wherein complete remission is defined as the absence of new and established pemphigus lesions.

In some embodiments, said compound is for use in methods comprising achieving complete remission of disease in 8% to 18% of the human patient population.

In some embodiments, said compound is for use in methods comprising achieving complete remission of disease in 13% of the human patient population.

In some embodiments, the human patient population is undergoing treatment for pemphigus vulgaris or pemphigus foliaceus.

In some embodiments, each member of the human patient population was characterized by relapsing pemphigus vulgaris or relapsing pemphigus foliaceus prior to the administration of PRN1008. In some embodiments, each member of the human patient population was characterized by relapsing pemphigus vulgaris prior to the administration of PRN1008.

In some embodiments, the first corticosteroid administered to a member of the human patient population and the second corticosteroid administered to the member of the human patient population are independently chosen from prednisone, prednisolone, and methylprednisolone. In some embodiments, the first corticosteroid administered to a member of the human patient population is the same as the second corticosteroid administered to the member of the human patient population. In some embodiments, the first corticosteroid administered to a member of the human patient population is not the same as the second corticosteroid administered to the member of the human patient population.

Also provided is compound (R)-2-[3-[4-amino-3-(2-fluoro-4-phenoxyphenyl)pyrazolo[3,4-d]pyrimidin-1-yl]piperidine-1-carbonyl]-4-methyl-4-[4-(oxetan-3-yl)piperazin-1-yl]pent-2-enenitrile (PRN1008) for use in a method for achieving complete remission of disease in 13% of a human patient population undergoing treatment for pemphigus vulgaris (PV) or pemphigus or pemphigus foliaceus (PF) comprising administering to the patient population 400 mg of said compound once a day (QD) for 14 days, followed by escalating the dose of PRN1008 to 400 mg BID for 14 days, followed by further escalating the dose to 600 mg BID for 56 days, wherein throughout the administration of PRN1008 to the patient population, the patient population also administered a corticosteroid at a dose of less than or equal to 0.5 mg/kg/day (≤ 0.5 mg/kg/day); wherein prior to the 400 mg QD administration of PRN1008, the patient population has (a) relapsing PV; and (b) a pemphigus disease activity index (PDAI) skin score of 8-60 points, and is maintained on a low dose corticosteroid (LDCS) therapy that comprises administering a corticosteroid at a dose of ≤ 0.5 mg/kg/day; wherein complete remission means the absence new and established lesions from PV or PF.

Some embodiments of the present disclosure relate to compound (R)-2-[3-[4-amino-3-(2-fluoro-4-phenoxy-phenyl)pyrazolo[3,4-d]pyrimidin-1-yl]piperidine-1-carbonyl]-4-methyl-4-[4-(oxetan-3-yl)piperazin-1-yl]pent-2-enenitrile (PRN1008) for use in methods for achieving complete remission of disease in 30% to 50% of a human patient population undergoing treatment for pemphigus comprising:
administering to each member of the human patient population a dose of 400 mg of said compound once a day (QD) for 14 days, optionally in combination with a first corticosteroid at a dose of less than or equal to 0.5 mg/kg/day (≤ 0.5 mg/kg/day), followed by a dose of 400 mg of PRN1008 twice a day (BID) for 14 days in combination with the first corticosteroid at a dose of less than or equal to 0.5 mg/kg/day (≤ 0.5 mg/kg/day), further followed by a dose of 600 mg of PRN1008 twice a day (BID) for 140 days in combination with the first corticosteroid at a dose of less than or equal to 0.5 mg/kg/day (≤ 0.5 mg/kg/day); and
subjecting each member of the human patient population to a 28 day post-treatment period following the administration of PRN1008 during which no PRN1008 or corticosteroid is administered, wherein each member of the human patient population was characterized by:
   relapsing pemphigus;
   a pemphigus disease activity index (PDAI) skin score of 8-60 points; and
   a maintenance dose of less than or equal to 0.5 mg/kg/day (≤ 0.5 mg/kg/day) of a second corticosteroid,
   prior to the administration of PRN1008,
   wherein complete remission is defined as the absence of new and established pemphigus lesions.

In some embodiments, said compound is for use in methods comprising achieving complete remission of disease in 35% to 45% of the human patient population.

In some embodiments, said compound is for use in methods comprising achieving complete remission of disease in 40% of the human patient population.

In some embodiments, the human patient population is undergoing treatment for pemphigus vulgaris or pemphigus foliaceus.

In some embodiments, the first corticosteroid administered to a member of the human patient population and the second corticosteroid administered to the member of the human patient population are independently chosen from prednisone, prednisolone, and methylprednisolone. In some embodiments, the first corticosteroid administered to a member of the human patient population is the same as the second corticosteroid administered to the member of the human patient population. In some embodiments, the first corticosteroid administered to a member of the human patient population is not the same as the second corticosteroid administered to the member of the human patient population.

Also provided is compound (R)-2-[3-[4-amino-3-(2-fluoro-4-phenoxyphenyl)pyrazolo[3,4-d]pyrimidin-1-yl]piperidine-1-carbonyl]-4-methyl-4-[4-(oxetan-3-yl)piperazin-1-yl]pent-2-enenitrile (PRN1008) for use in a method for achieving complete remission of disease in 40% of a human patient population undergoing treatment for pemphigus vulgaris (PV) or pemphigus or pemphigus foliaceus (PF) comprising administering to the patient population 400 mg of said compound once a day (QD) for 14 days, followed by escalating the dose of PRN1008 to 400 mg BID for 14 days, followed by further escalating the dose to 600 mg BID for 140 days, and thereafter subjecting the patient population to post-treatment follow-up for 28 days, during which follow-up, the patient population is not administered any PRN1008 or corticosteroid; wherein throughout the administration of PRN1008 to the patient population, the patient population also administered a corticosteroid at a dose of less than or equal to 0.5 mg/kg/day (≤ 0.5 mg/kg/day); wherein prior to the 400 mg QD administration of PRN1008, the patient population has (a) relapsing PV; and (b) a pemphigus disease activity index (PDAI) skin score of 8-60 points, and is maintained on a low dose corticosteroid (LDCS) therapy that comprises administering a corticosteroid at a dose of ≤ 0.5 mg/kg/day; wherein complete remission means the absence of new and established lesions from PV or PF.

Some embodiments of the present disclosure relate to compound (R)-2-[3-[4-amino-3-(2-fluoro-4-phenoxy-phenyl)pyrazolo[3,4-d]pyrimidin-1-yl]piperidine-1-carbonyl]-4-methyl-4-[4-(oxetan-3-yl)piperazin-1-yl]pent-2-enenitrile (PRN1008) for use in methods for treating pemphigus in a human patient, wherein the human patient is characterized by relapsing pemphigus prior to the administration of PRN1008, comprising:
administering to the human patient a dose of 400 mg of said compound once a day, optionally in combination with a first corticosteroid at a dose of less than or equal to 0.5 mg/kg/day (≤ 0.5 mg/kg/day);
assessing whether the human patient is achieving control of disease activity and/or reaching the end of consolidation period during week 3 and escalating the dose of PRN1008 to 400 mg BID if the human patient is not achieving control of disease activity and/or not reaching the end of consolidation period during week 3;
assessing whether the human patient is achieving control of disease activity and/or reaching the end of consolidation period during week 5 and escalating the dose of PRN1008 to 600 mg BID if the human patient is not achieving control of disease activity and/or not reaching the end of consolidation period during week 5, wherein:
   control of disease activity (CDA) is defined as a visit for a medical checkup at which new pemphigus lesions cease to form and established pemphigus lesions begin to heal;
   end of consolidation phase is defined as a medical visit at which no new pemphigus lesion has developed for a minimum of 2 weeks and a majority of established pemphigus lesions have healed.

The human patient is characterized by relapsing pemphigus prior to the administration of PRN1008. In some embodiments, the human patient is characterized by relapsing pemphigus vulgaris prior to the administration of PRN1008. In some embodiments, the human patient is characterized by relapsing pemphigus foliaceus prior to the administration of PRN1008.

In some embodiments, the human patient is characterized by a pemphigus disease activity index (PDAI) skin score of 8-60 points prior to the administration of PRN1008.

In some embodiments, the human patient is characterized by a maintenance dose of less than or equal to 0.5 mg/kg/day (≤ 0.5 mg/kg/day) of a second corticosteroid prior to the administration of PRN1008.

In some embodiments, the human patient is characterized by:
a pemphigus disease activity index (PDAI) skin score of 8-60 points; and
a maintenance dose of less than or equal to 0.5 mg/kg/day (≤ 0.5 mg/kg/day) of a second corticosteroid,
prior to the administration of PRN1008.

In some embodiments, the first corticosteroid administered to a member of the human patient population and the second corticosteroid administered to the member of the human patient population are independently chosen from prednisone, prednisolone, and methylprednisolone. In some embodiments, the first corticosteroid administered to a member of the human patient population is the same as the second corticosteroid administered to the member of the human patient population. In some embodiments, the first corticosteroid administered to a member of the human patient population is not the same as the second corticosteroid administered to the member of the human patient population.

In some embodiments, PRN1008 is administered to the patient for 25 weeks.

In some embodiments, end of consolidation phase is defined as a medical visit at which no new pemphigus lesion has developed for a minimum of 2 weeks and at least 50% of established pemphigus lesions have healed. In some embodiments, end of consolidation phase is defined as a medical visit at which no new pemphigus lesion has developed for a minimum of 2 weeks and at least 60% of established pemphigus lesions have healed. In some embodiments, end of consolidation phase is defined as a medical visit at which no new pemphigus lesion has developed for a minimum of 2 weeks and at least 70% of established pemphigus lesions have healed. In some embodiments, end of consolidation phase is defined as a medical visit at which no new pemphigus lesion has developed for a minimum of 2 weeks and at least 80% of established pemphigus lesions have healed. In some embodiments, end of consolidation phase is defined as a medical visit at which no new pemphigus lesion has developed for a minimum of 2 weeks and at least 90% of established pemphigus lesions have healed.

Also provided is compound (R)-2-[3-[4-amino-3-(2-fluoro-4-phenoxyphenyl)pyrazolo[3,4-d]pyrimidin-1-yl]piperidine-1-carbonyl]-4-methyl-4-[4-(oxetan-3-yl)piperazin-1-yl]pent-2-enenitrile (PRN1008) for use in a method for treating pemphigus vulgaris (PV) or pemphigus or pemphigus foliaceus (PF) in a patient population comprising administering to the population a dosing regimen as follows:
administering said compound at 400 mg QD starting dose (at week 1) with dose escalation allowed at week 3 to 400 mg BID and at week 5 to 600 mg BID, wherein the dosing period ends at week 25;
wherein the condition for dose escalation is either (a) the patient not achieving control of disease activity (CDA) or (b) not reaching end of consolidation phase (ECP);
wherein control of disease activity is defined as the visit at which new lesions from PV or PF cease to form and established lesions from PV or PF begin to heal;
wherein end of consolidation phase is defined as the visit at which no new lesions from PV or PF have developed for a minimum of 2 weeks and a majority of established lesions from PV or PF have healed;
wherein throughout the administration of PRN1008 to the patient population, the patient population also administered a corticosteroid at a dose of less than or equal to 0.5 mg/kg/day (≤ 0.5 mg/kg/day); wherein prior to the 400 mg QD administration of PRN1008, the patient population has (a) relapsing PV; and (b) a pemphigus disease activity index (PDAI) skin score of 8-60 points, and is maintained on a low dose corticosteroid (LDCS) therapy that comprises administering a corticosteroid at a dose of ≤ 0.5 mg/kg/day.

Some embodiments of the present disclosure relate to compound (R)-2-[3-[4-amino-3-(2-fluoro-4-phenoxy-phenyl)pyrazolo[3,4-d]pyrimidin-1-yl]piperidine-1-carbonyl]-4-methyl-4-[4-(oxetan-3-yl)piperazin-1-yl]pent-2-enenitrile (PRN1008) for use in methods of treating pemphigus in a human patient comprising administering to the human patient a dose of 400 mg of said compound twice a day (BID) for 168 days, optionally in combination with a first corticosteroid at a dose of less than or equal to 0.5 mg/kg/day (≤ 0.5 mg/kg/day), wherein the human patient is characterized by relapsing pemphigus prior to the administration of PRN1008.

In some embodiments, the human patient is characterized by:
a pemphigus disease activity index (PDAI) skin score of 8-60 points,
a maintenance dose of less than or equal to 0.5 mg/kg/day (≤ 0.5 mg/kg/day) of a second corticosteroid,
prior to the administration of PRN1008.

In some embodiments, said compound is for use in methods comprising treating pemphigus vulgaris. In some embodiments, said compound is for use in methods comprising treating pemphigus foliaceus.

In some embodiments, the human patient is characterized by relapsing pemphigus vulgaris prior to the administration of PRN1008. In some embodiments, the human patient is characterized by relapsing pemphigus foliaceus prior to the administration of PRN1008.

In some embodiments, said compound is for use in methods comprising treating pemphigus vulgaris and the human patient is characterized by relapsing pemphigus vulgaris prior to the administration of PRN1008. In some embodiments, said compound is for use in methods comprising treating pemphigus foliaceus and the human patient is characterized by relapsing pemphigus foliaceus prior to the administration of PRN1008.

In some embodiments, the first corticosteroid administered to a member of the human patient population and the second corticosteroid administered to the member of the human patient population are independently chosen from prednisone, prednisolone, and methylprednisolone. In some embodiments, the first corticosteroid administered to a member of the human patient population is the same as the second corticosteroid administered to the member of the human patient population. In some embodiments, the first corticosteroid administered to a member of the human patient population is not the same as the second corticosteroid administered to the member of the human patient population.

Also provided is compound (R)-2-[3-[4-amino-3-(2-fluoro-4-phenoxyphenyl)pyrazolo[3,4-d]pyrimidin-1-yl]piperidine-1-carbonyl]-4-methyl-4-[4-(oxetan-3-yl)piperazin-1-yl]pent-2-enenitrile (PRN1008) for use in a method of treating a human patient afflicted with relapsing pemphigus vulgaris (PV) or relapsing pemphigus foliaceus (PF) comprising:
administering to the patient a dose of 400 mg of said compound twice a day (BID) for 168 days, wherein throughout the administration of PRN1008, the patient is also administered a corticosteroid at a dose of less than or equal to 0.5 mg/kg/day (≤ 0.5 mg/kg/day); wherein prior to the 400 mg BID administration of PRN1008, the patient has (a) relapsing PV; and (b) a pemphigus disease activity index (PDAI) skin score of 8-60 points, and is maintained on a low dose corticosteroid (LDCS) therapy that comprises administering a corticosteroid at a dose of ≤ 0.5 mg/kg/day.

In some embodiments the corticosteroid is prednisone, prednisolone, or methylprednisolone. In some embodiments PRN1008 comprises a mixture of (E) and (Z) isomers of (R)-2-[3-[4-amino-3-(2-fluoro-4-phenoxy-phenyl)pyrazolo[3,4-d]pyrimidin-1-yl]piperidine-1-carbonyl]-4-methyl-4-[4-(oxetan-3-yl)piperazin-1-yl]pent-2-enenitrile. In some embodiments after the administration of PRN1008, the patient has a PDAI score of 1 (near-clear skin).

The present disclosure also provides compound (R)-2-[3-[4-amino-3-(2-fluoro-4-phenoxy-phenyl)pyrazolo[3,4-d]pyrimidin-1-yl]piperidine-1-carbonyl]-4-methyl-4-[4-(oxetan-3-yl)piperazin-1-yl]pent-2-enenitrile (PRN1008) for use in a method of treating a human patient afflicted with relapsing pemphigus vulgaris (PV) or relapsing pemphigus foliaceus (PF) comprising:
administering a dose of 400 mg of said compound twice a day (BID) for at least 14 days.

In some embodiments, said compound is for use in a method further comprising administering to the patient a corticosteroid at a dose of less than or equal to 0.5 mg/kg/day (≤ 0.5 mg/kg/day). In some embodiments, the 400 mg BID dose is administered for a maximum of 84 days. In some embodiments, the 400 mg BID dose is administered for 14 days. In some embodiments, the 400 mg BID dose is administered for 28 days. Some embodiments further comprise escalating the 400 mg BID dose to 500 mg BID after a minimum of 14-28 days. In some embodiments, the 400 mg BID dose is escalated to 500 mg BID after 33 days. Some embodiments, further comprise escalating the 400 mg BID dose to 600 mg BID after a minimum of 14-28 days. In some embodiments, the 400 mg BID dose is escalated to 600 mg BID after 22 days. In some embodiments, the 400 mg BID dose is escalated to 600 mg BID after 56 days. Some embodiments further comprise escalating the 500 mg BID dose to 600 mg BID after 14-28 days. In some embodiments, prior to the administration of the 400 mg BID dose, the patient is maintained on a low dose corticosteroid (LDCS) therapy that comprises administering a corticosteroid at a dose of ≤ 0.5 mg/kg/day. In some embodiments, prior to the administration of the 400 mg BID dose, the patient has (a) relapsing PV; and (b) a pemphigus disease activity index (PDAI) skin score of 8-45 points. In some embodiments, the corticosteroid is prednisone, prednisolone, or methylprednisolone. In some embodiments, PRN1008 comprises a mixture of (E) and (Z) isomers of (R)-2-[3-[4-amino-3-(2-fluoro-4-phenoxy-phenyl)pyrazolo[3,4-d]pyrimidin-1-yl]piperidine-1-carbonyl]-4-methyl-4-[4-(oxetan-3-yl)piperazin-1-yl]pent-2-enenitrile. In some embodiments, after the administration of PRN1008, the patient has a PDAI score of 1 (near-clear skin).

In some embodiments, said compound is for use in methods comprising:
administering to the human patient a first dose of 400 mg of PRN1008 once a day (QD) for 14 days; and
administering to the human patient a second dose of 400 mg of PRN1008 twice a day (BID) for at least 14 days following the administration of the first dose.

In some embodiments, the human patient is administered a second dose of 400 mg of PRN1008 BID for 14 to 154 days following the administration of the first dose.

In some embodiments, PRN1008 is administered to the human patient for at most 168 days.

In some embodiments, the methods comprise:
administering to the human patient a first dose of 400 mg of PRN1008 once a day (QD) for 14 days; and
administering to the human patient a second dose of 400 mg of PRN1008 twice a day (BID) for 14 days following the administration of the first dose; and
administering to the human patient a third dose of 600 mg of PRN1008 twice a day (BID) following the administration of the second dose.

In some embodiments, the human patient is administered a third dose of 600 mg of PRN1008 BID for at most 140 days following the administration of the second dose. In some embodiments, the human patient is administered a third dose of 600 mg of PRN1008 BID for 56 days following the administration of the second dose.

In some embodiments, PRN1008 is administered to the human patient for at most 168 days.

Some embodiments of the present disclosure relate to compound (R)-2-[3-[4-amino-3-(2-fluoro-4-phenoxy-phenyl)pyrazolo[3,4-d]pyrimidin-1-yl]piperidine-1-carbonyl]-4-methyl-4-[4-(oxetan-3-yl)piperazin-1-yl]pent-2-enenitrile (PRN1008) for use in methods for achieving a primary endpoint in 17% to 37% of a human patient population undergoing treatment for pemphigus comprising:
administering to each member of the human patient population a dose of 400 mg of said compound twice a day (BID) for 14 days, optionally in combination with a first corticosteroid at a dose of less than or equal to 0.5 mg/kg/day (≤ 0.5 mg/kg/day), wherein each member of the human patient population was characterized by:
relapsing pemphigus;
a pemphigus disease activity index (PDAI) skin score of 8-45 points; and
a maintenance dose of less than or equal to 0.5 mg/kg/day (≤ 0.5 mg/kg/day) of a second corticosteroid,
prior to the administration of PRN1008,
wherein the primary endpoint comprises control of disease activity (CDA) defined as a visit for a medical checkup at which new pemphigus lesions cease to form and established pemphigus lesions begin to heal.

In some embodiments, said compound is for use in methods comprising achieving complete remission of disease in 22% to 32% of the human patient population.

In some embodiments, said compound is for use in methods comprising achieving complete remission of disease in 27% of the human patient population.

In some embodiments, the human patient population is undergoing treatment for pemphigus vulgaris or pemphigus foliaceus.

In some embodiments, each member of the human patient population is undergoing treatment for pemphigus vulgaris or pemphigus foliaceus.

In some embodiments, each member of the human patient population was characterized by relapsing pemphigus vulgaris or relapsing pemphigus foliaceus prior to the administration of PRN1008.

In some embodiments, the first corticosteroid administered to a member of the human patient population and the second corticosteroid administered to the member of the human patient population are independently chosen from prednisone, prednisolone, and methylprednisolone. In some embodiments, the first corticosteroid administered to a member of the human patient population is the same as the second corticosteroid administered to the member of the human patient population. In some embodiments, the first corticosteroid administered to a member of the human patient population is not the same as the second corticosteroid administered to the member of the human patient population.

Also provided is compound (R)-2-[3-[4-amino-3-(2-fluoro-4-phenoxyphenyl)pyrazolo[3,4-d]pyrimidin-1-yl]piperidine-1-carbonyl]-4-methyl-4-[4-(oxetan-3-yl)piperazin-1-yl]pent-2-enenitrile (PRN1008) for use in a method for achieving a primary endpoint in 27% of a human patient population undergoing treatment for pemphigus vulgaris (PV) or pemphigus or pemphigus foliaceus (PF) comprising administering to the patient population 400 mg of said compound twice a day (BID) for 14 days in combination with a corticosteroid at a dose of less than or equal to 0.5 mg/kg/day (≤ 0.5 mg/kg/day), wherein prior to the 400 mg BID administration of PRN1008, the patient population has (a) relapsing PV; and (b) a pemphigus disease activity index (PDAI) skin score of 8-45 points, and is maintained on a low dose corticosteroid (LDCS) therapy that comprises administering a corticosteroid at a dose of ≤ 0.5 mg/kg/day; wherein the primary endpoint comprises control of disease activity (CDA) defined as a visit for medical checkup at which new lesions from PV or PF cease to form and established lesions from PV and PF begin to heal.

Some embodiments of the present disclosure relate to compound (R)-2-[3-[4-amino-3-(2-fluoro-4-phenoxy-phenyl)pyrazolo[3,4-d]pyrimidin-1-yl]piperidine-1-carbonyl]-4-methyl-4-[4-(oxetan-3-yl)piperazin-1-yl]pent-2-enenitrile (PRN1008) for use in methods for achieving a primary endpoint in 44% to 64% of a human patient population undergoing treatment for pemphigus comprising:
administering to each member of the human patient population a dose of 400 mg of said compound twice a day (BID) for 28 days, optionally in combination with a first corticosteroid at a dose of less than or equal to 0.5 mg/kg/day (≤ 0.5 mg/kg/day), wherein each member of the human patient population was characterized by:
relapsing pemphigus;
a pemphigus disease activity index (PDAI) skin score of 8-45 points; and
a maintenance dose of less than or equal to 0.5 mg/kg/day (≤ 0.5 mg/kg/day) of a second corticosteroid,
prior to the administration of PRN1008,
wherein the primary endpoint comprises control of disease activity (CDA) defined as a visit for a medical checkup at which new pemphigus lesions cease to form and established pemphigus lesions begin to heal.

In some embodiments, said compound is for use in methods comprising achieving complete remission of disease in 49% to 59% of the human patient population.

In some embodiments, said compound is for use in methods comprising achieving complete remission of disease in 54% of the human patient population.

In some embodiments, the human patient population is undergoing treatment for pemphigus vulgaris or pemphigus foliaceus.

In some embodiments, each member of the human patient population is undergoing treatment for pemphigus vulgaris or pemphigus foliaceus.

In some embodiments, each member of the human patient population was characterized by relapsing pemphigus vulgaris or relapsing pemphigus foliaceus prior to the administration of PRN1008.

In some embodiments, the first corticosteroid administered to a member of the human patient population and the second corticosteroid administered to the member of the human patient population are independently chosen from prednisone, prednisolone, and methylprednisolone. In some embodiments, the first corticosteroid administered to a member of the human patient population is the same as the second corticosteroid administered to the member of the human patient population. In some embodiments, the first corticosteroid administered to a member of the human patient population is not the same as the second corticosteroid administered to the member of the human patient population.

Some embodiments of the present disclosure relate to compound (R)-2-[3-[4-amino-3-(2-fluoro-4-phenoxy-phenyl)pyrazolo[3,4-d]pyrimidin-1-yl]piperidine-1-carbonyl]-4-methyl-4-[4-(oxetan-3-yl)piperazin-1-yl]pent-2-enenitrile (PRN1008) for use in a method for achieving a primary endpoint in 54% of a human patient population undergoing treatment for pemphigus vulgaris (PV) or pemphigus or pemphigus foliaceus (PF) comprises administering to the patient population 400 mg of said compound twice a day (BID) for 28 days in combination with a corticosteroid at a dose of less than or equal to 0.5 mg/kg/day (≤ 0.5 mg/kg/day), wherein prior to the 400 mg BID administration of PRN1008, the patient population has (a) relapsing PV; and (b) a pemphigus disease activity index (PDAI) skin score of 8-45 points, and is maintained on a low dose corticosteroid (LDCS) therapy that comprises administering a corticosteroid at a dose of ≤ 0.5 mg/kg/day; wherein the primary endpoint comprises control of disease activity (CDA) defined as a visit for medical checkup at which new lesions from PV or PF cease to form and established lesions from PV and PF begin to heal.

Some embodiments of the present disclosure relate to compound (R)-2-[3-[4-amino-3-(2-fluoro-4-phenoxy-phenyl)pyrazolo[3,4-d]pyrimidin-1-yl]piperidine-1-carbonyl]-4-methyl-4-[4-(oxetan-3-yl)piperazin-1-yl]pent-2-enenitrile (PRN1008) for use in methods for achieving a primary endpoint in 63% to 83% of a human patient population undergoing treatment for pemphigus comprising:
administering to each member of the human patient population a dose of 400 mg of said compound twice a day (BID) for 84 days, optionally in combination with a first corticosteroid at a dose of less than or equal to 0.5 mg/kg/day (≤ 0.5 mg/kg/day), wherein each member of the human patient population was characterized by:
relapsing pemphigus;
a pemphigus disease activity index (PDAI) skin score of 8-45 points; and
a maintenance dose of less than or equal to 0.5 mg/kg/day (≤ 0.5 mg/kg/day) of a second corticosteroid,
prior to the administration of PRN1008,
wherein the primary endpoint comprises control of disease activity (CDA) defined as a visit for a medical checkup at which new pemphigus lesions cease to form and established pemphigus lesions begin to heal.

In some embodiments, said compound is for use in methods comprising achieving the primary endpoint in 68% to 78% of the human patient population.

In some embodiments, said compound is for use in methods comprising achieving the primary endpoint in 73% of the human patient population.

In some embodiments, the human patient population is undergoing treatment for pemphigus vulgaris or pemphigus foliaceus.

In some embodiments, each member of the human patient population is undergoing treatment for pemphigus vulgaris or pemphigus foliaceus.

In some embodiments, each member of the human patient population was characterized by relapsing pemphigus vulgaris or relapsing pemphigus foliaceus prior to the administration of PRN1008. In some embodiments, each member of the human patient population was characterized by relapsing pemphigus vulgaris.

In some embodiments, the first corticosteroid administered to a member of the human patient population and the second corticosteroid administered to the member of the human patient population are independently chosen from prednisone, prednisolone, and methylprednisolone. In some embodiments, the first corticosteroid administered to a member of the human patient population is the same as the second corticosteroid administered to the member of the human patient population. In some embodiments, the first corticosteroid administered to a member of the human patient population is not the same as the second corticosteroid administered to the member of the human patient population.

Also provided is compound (R)-2-[3-[4-amino-3-(2-fluoro-4-phenoxyphenyl)pyrazolo[3,4-d]pyrimidin-1-yl]piperidine-1-carbonyl]-4-methyl-4-[4-(oxetan-3-yl)piperazin-1-yl]pent-2-enenitrile (PRN1008) for use in a method for achieving a primary endpoint in 73% of a human patient population undergoing treatment for pemphigus vulgaris (PV) or pemphigus or pemphigus foliaceus (PF) comprising administering to the patient population 400 mg of said compound twice a day (BID) for 84 days in combination with a corticosteroid at a dose of less than or equal to 0.5 mg/kg/day (≤ 0.5 mg/kg/day), wherein prior to the 400 mg BID administration of PRN1008, the patient population has (a) relapsing PV; and (b) a pemphigus disease activity index (PDAI) skin score of 8-45 points, and is maintained on a low dose corticosteroid (LDCS) therapy that comprises administering a corticosteroid at a dose of ≤ 0.5 mg/kg/day; wherein the primary endpoint comprises control of disease activity (CDA) defined as a visit for medical checkup at which new lesions from PV or PF cease to form and established lesions from PV and PF begin to heal.

Some embodiments of the present disclosure relate to compound (R)-2-[3-[4-amino-3-(2-fluoro-4-phenoxy-phenyl)pyrazolo[3,4-d]pyrimidin-1-yl]piperidine-1-carbonyl]-4-methyl-4-[4-(oxetan-3-yl)piperazin-1-yl]pent-2-enenitrile (PRN1008) for use in methods for achieving complete remission of disease in 6% to 26% of a human patient population undergoing treatment for pemphigus comprising:
administering to each member of the human patient population a dose of 400 mg of said compound twice a day (BID) for 84 days, optionally in combination with a first corticosteroid at a dose of less than or equal to 0.5 mg/kg/day (≤ 0.5 mg/kg/day), wherein each member of the human patient population was characterized by:
relapsing pemphigus;
a pemphigus disease activity index (PDAI) skin score of 8-45 points; and
a maintenance dose of less than or equal to 0.5 mg/kg/day (≤ 0.5 mg/kg/day) of a second corticosteroid,
prior to the administration of PRN1008,
wherein complete remission is defined as the absence of new and established pemphigus lesions.

In some embodiments, said compound is for use in methods comprising achieving complete remission of disease in 11% to 21% of the human patient population.

In some embodiments, said compound is for use in methods comprising achieving complete remission of disease in 15% to 17% of the human patient population.

In some embodiments, said compound is for use in methods comprising achieving complete remission of disease in 15% or 17% of the human patient population. In some embodiments, said compound is for use in methods comprising achieving complete remission of disease in 15% of the human patient population. In some embodiments, said compound is for use in methods comprising achieving complete remission of disease in 17% of the human patient population.

In some embodiments, the human patient population is undergoing treatment for pemphigus vulgaris or pemphigus foliaceus.

In some embodiments, each member of the human patient population is undergoing treatment for pemphigus vulgaris or pemphigus foliaceus.

In some embodiments, each member of the human patient population was characterized by relapsing pemphigus vulgaris or relapsing pemphigus foliaceus prior to the administration of PRN1008. In some embodiments, each member of the human patient population was characterized by relapsing pemphigus vulgaris.

In some embodiments, the first corticosteroid administered to a member of the human patient population and the second corticosteroid administered to the member of the human patient population are independently chosen from prednisone, prednisolone, and methylprednisolone. In some embodiments, the first corticosteroid administered to a member of the human patient population is the same as the second corticosteroid administered to the member of the human patient population. In some embodiments, the first corticosteroid administered to a member of the human patient population is not the same as the second corticosteroid administered to the member of the human patient population.

Also provided is compound (R)-2-[3-[4-amino-3-(2-fluoro-4-phenoxyphenyl)pyrazolo[3,4-d]pyrimidin-1-yl]piperidine-1-carbonyl]-4-methyl-4-[4-(oxetan-3-yl)piperazin-1-yl]pent-2-enenitrile (PRN1008) for use in a method for achieving complete remission of disease in 17% of a human patient population undergoing treatment for pemphigus vulgaris (PV) or pemphigus or pemphigus foliaceus (PF) comprising administering to the patient population 400 mg of said compound twice a day (BID) for 84 days, wherein prior to the 400 mg BID administration of PRN1008, the patient population has (a) relapsing PV; and (b) a pemphigus disease activity index (PDAI) skin score of 8-45 points, and is maintained on a low dose corticosteroid (LDCS) therapy that comprises administering a corticosteroid at a dose of ≤ 0.5 mg/kg/day; wherein complete remission means the absence of new and established lesions from PV or PF.

Also provided is compound (R)-2-[3-[4-amino-3-(2-fluoro-4-phenoxyphenyl)pyrazolo[3,4-d]pyrimidin-1-yl]piperidine-1-carbonyl]-4-methyl-4-[4-(oxetan-3-yl)piperazin-1-yl]pent-2-enenitrile (PRN1008) for use in a method for achieving complete remission of disease in 15% to 17% of a human patient population undergoing treatment for pemphigus vulgaris (PV) or pemphigus or pemphigus foliaceus (PF) comprising administering to the patient population 400 mg of said compound twice a day (BID) for 84 days, wherein prior to the 400 mg BID administration of PRN1008, the patient population has (a) relapsing PV; and (b) a pemphigus disease activity index (PDAI) skin score of 8-45 points, and is maintained on a low dose corticosteroid (LDCS) therapy that comprises administering a corticosteroid at a dose of ≤ 0.5 mg/kg/day; wherein complete remission means the absence of new and established lesions from PV or PF.

Also provided is compound (R)-2-[3-[4-amino-3-(2-fluoro-4-phenoxyphenyl)pyrazolo[3,4-d]pyrimidin-1-yl]piperidine-1-carbonyl]-4-methyl-4-[4-(oxetan-3-yl)piperazin-1-yl]pent-2-enenitrile (PRN1008) for use in a method for achieving complete remission of disease in 15% or 17% of a human patient population undergoing treatment for pemphigus vulgaris (PV) or pemphigus or pemphigus foliaceus (PF) comprising administering to the patient population 400 mg of said compound twice a day (BID) for 84 days, wherein prior to the 400 mg BID administration of PRN1008, the patient population has (a) relapsing PV; and (b) a pemphigus disease activity index (PDAI) skin score of 8-45 points, and is maintained on a low dose corticosteroid (LDCS) therapy that comprises administering a corticosteroid at a dose of ≤ 0.5 mg/kg/day; wherein complete remission means the absence of new and established lesions from PV or PF.

Some embodiments of the present disclosure relate to compound (R)-2-[3-[4-amino-3-(2-fluoro-4-phenoxy-phenyl)pyrazolo[3,4-d]pyrimidin-1-yl]piperidine-1-carbonyl]-4-methyl-4-[4-(oxetan-3-yl)piperazin-1-yl]pent-2-enenitrile (PRN1008) for use in methods for achieving complete remission of disease in 14% to 34% of a human patient population undergoing treatment for pemphigus comprising:
administering to each member of the human patient population a dose of 400 mg of said compound twice a day (BID) for 84 days, optionally in combination with a first corticosteroid at a dose of less than or equal to 0.5 mg/kg/day (≤ 0.5 mg/kg/day); and
subjecting each member of the patient population to an 84 day post-treatment period following the administration of PRN1008 during which no PRN1008 or corticosteroid is administered, wherein each member of the human patient population was characterized by:
   relapsing pemphigus;
   a pemphigus disease activity index (PDAI) skin score of 8-45 points; and
   a maintenance dose of less than or equal to 0.5 mg/kg/day (≤ 0.5 mg/kg/day) of a second corticosteroid,
   prior to the administration of PRN1008,
   wherein complete remission is defined as the absence of new and established pemphigus lesions.

In some embodiments, said compound is for use in methods comprising achieving the primary endpoint in 19% to 29% of the human patient population.

In some embodiments, said compound is for use in methods comprising achieving the primary endpoint in 23% to 25% of the human patient population.

In some embodiments, said compound is for use in methods comprising achieving the primary endpoint in 23% or 25% of the human patient population. In some embodiments, said compound is for use in methods comprising achieving the primary endpoint in 23% of the human patient population. In some embodiments, said compound is for use in methods comprising achieving the primary endpoint in 25% of the human patient population.

In some embodiments, the human patient population is undergoing treatment for pemphigus vulgaris or pemphigus foliaceus.

In some embodiments, each member of the human patient population is undergoing treatment for pemphigus vulgaris or pemphigus foliaceus.

In some embodiments, each member of the human patient population was characterized by relapsing pemphigus vulgaris or relapsing pemphigus foliaceus prior to the administration of PRN1008. In some embodiments, each member of the human patient population was characterized by relapsing pemphigus vulgaris.

In some embodiments, the first corticosteroid administered to a member of the human patient population and the second corticosteroid administered to the member of the human patient population are independently chosen from prednisone, prednisolone, and methylprednisolone. In some embodiments, the first corticosteroid administered to a member of the human patient population is the same as the second corticosteroid administered to the member of the human patient population. In some embodiments, the first corticosteroid administered to a member of the human patient population is not the same as the second corticosteroid administered to the member of the human patient population.

Also provided is compound (R)-2-[3-[4-amino-3-(2-fluoro-4-phenoxyphenyl)pyrazolo[3,4-d]pyrimidin-1-yl]piperidine-1-carbonyl]-4-methyl-4-[4-(oxetan-3-yl)piperazin-1-yl]pent-2-enenitrile (PRN1008) for use in a method for achieving complete remission of disease in 23% or 25% of a human patient population undergoing treatment for pemphigus vulgaris (PV) or pemphigus or pemphigus foliaceus (PF) comprising administering to the patient population 400 mg of said compound twice a day (BID) for 84 days, and thereafter subjecting the patient population to post-treatment follow-up for 84 days, during which follow-up, the patient population is not administered any PRN1008 or corticosteroid; wherein throughout the administration of PRN1008 to the patient population, the patient population also administered a corticosteroid at a dose of less than or equal to 0.5 mg/kg/day (≤ 0.5 mg/kg/day); wherein prior to the 400 mg QD administration of PRN1008, the patient population has (a) relapsing PV; and (b) a pemphigus disease activity index (PDAI) skin score of 8-45 points, and is maintained on a low dose corticosteroid (LDCS) therapy that comprises administering a corticosteroid at a dose of ≤ 0.5 mg/kg/day; wherein complete remission means the absence of new and established lesions from PV or PF. In some embodiments, complete remission of disease is achieved in 23% of a human patient population. In some embodiments, complete remission of disease is achieved in 25% of a human patient population.

Compound(R)-2-[3-[4-amino-3-(2-fluoro-4-phenoxy-phenyl)pyrazolo[3,4-d]pyrimidin-1-yl]piperidine-1-carbonyl]-4-methyl-4-[4-(oxetan-3-yl)piperazin-1-yl]pent-2-enenitrile (PRN1008) for use in a method for treating pemphigus vulgaris (PV) or pemphigus or pemphigus foliaceus (PF) in a patient population comprising administering to the population a dosing regimen as follows:
administering said compound at 400 mg QD starting dose (at week 1) with dose escalation allowed at week 3 to 400 mg BID and at week 5 to 600 mg BID, wherein the dosing period ends at week 25;
wherein the condition for dose escalation is either (a) the patient not achieving control of disease activity (CDA) or (b) not reaching end of consolidation phase (ECP);
wherein control of disease activity is defined as the visit at which new lesions from PV or PF cease to form and established lesions from PV or PF begin to heal;
wherein end of consolidation phase is defined as the visit at which no new lesions from PV or PF have developed for a minimum of 2 weeks and a majority of established lesions from PV or PF have healed;
wherein throughout the administration of PRN1008 to the patient population, the patient population also administered a corticosteroid at a dose of less than or equal to 0.5 mg/kg/day (≤ 0.5 mg/kg/day); wherein prior to the 400 mg QD administration of PRN1008, the patient population has (a) relapsing PV; and (b) a pemphigus disease activity index (PDAI) skin score of 8-60 points, and is maintained on a low dose corticosteroid (LDCS) therapy that comprises administering a corticosteroid at a dose of ≤ 0.5 mg/kg/day.

Also provided is compound (R)-2-[3-[4-amino-3-(2-fluoro-4-phenoxyphenyl)pyrazolo[3,4-d]pyrimidin-1-yl]piperidine-1-carbonyl]-4-methyl-4-[4-(oxetan-3-yl)piperazin-1-yl]pent-2-enenitrile (PRN1008) for use in a method of treating a human patient afflicted with pemphigus vulgaris (PV) or pemphigus foliaceus (PF) comprising:
administering to the patient a dose of 400 mg of said compound twice a day (BID) for 168 days, wherein throughout the administration of PRN1008, the patient is also administered a corticosteroid at a dose of less than or equal to 0.5 mg/kg/day (≤ 0.5 mg/kg/day); wherein prior to the 400 mg BID administration of PRN1008, the patient has (a) relapsing PV; and (b) a pemphigus disease activity index (PDAI) skin score of 8-60 points, and is maintained on a low dose corticosteroid (LDCS) therapy that comprises administering a corticosteroid at a dose of ≤ 0.5 mg/kg/day.

Some embodiments of the present disclosure relate to compound (R)-2-[3-[4-amino-3-(2-fluoro-4-phenoxy-phenyl)pyrazolo[3,4-d]pyrimidin-1-yl]piperidine-1-carbonyl]-4-methyl-4-[4-(oxetan-3-yl)piperazin-1-yl]pent-2-enenitrile (PRN1008) for use in methods for treating pemphigus in a human patient, wherein the human patient is characterized by relapsing pemphigus prior to the administration of said compound, comprising:
administering to the human patient a dose of 400 mg of said compound twice a day, optionally in combination with a first corticosteroid at a dose of less than or equal to 0.5 mg/kg/day (≤ 0.5 mg/kg/day); and
assessing whether the human patient is achieving control of disease activity and/or reaching the end of consolidation period and escalating the dose of PRN1008 to 500 mg BID or 600 mg BID if the human patient is not achieving control of disease activity and/or not reaching the end of consolidation period, wherein:
   control of disease activity (CDA) is defined as a visit for a medical checkup at which new pemphigus lesions cease to form and established pemphigus lesions begin to heal;
   end of consolidation phase is defined as a medical visit at which no new pemphigus lesion has developed for a minimum of 2 weeks and a majority of established pemphigus lesions have healed.
In some embodiments, the human patient is characterized by:
   a pemphigus disease activity index (PDAI) skin score of 8-60 points; and
   a maintenance dose of less than or equal to 0.5 mg/kg/day (≤ 0.5 mg/kg/day) of a second corticosteroid,
   prior to the administration of PRN1008.

In some embodiments, said compound is for use in methods comprising administering PRN1008 for 13 weeks.

In some embodiments, the first corticosteroid administered to a member of the human patient population and the second corticosteroid administered to the member of the human patient population are independently chosen from prednisone, prednisolone, and methylprednisolone. In some embodiments, the first corticosteroid administered to a member of the human patient population is the same as the second corticosteroid administered to the member of the human patient population. In some embodiments, the first corticosteroid administered to a member of the human patient population is not the same as the second corticosteroid administered to the member of the human patient population.

Also provided is compound (R)-2-[3-[4-amino-3-(2-fluoro-4-phenoxyphenyl)pyrazolo[3,4-d]pyrimidin-1-yl]piperidine-1-carbonyl]-4-methyl-4-[4-(oxetan-3-yl)piperazin-1-yl]pent-2-enenitrile (PRN1008) for use in a method for treating pemphigus vulgaris (PV) or pemphigus or pemphigus foliaceus (PF) in a patient population comprising administering to the population a dosing regimen as follows:
administering (R)-2-[3-[4-amino-3-(2-fluoro-4-phenoxy-phenyl)pyrazolo[3,4-d]pyrimidin-1-yl]piperidine-1-carbonyl]-4-methyl-4-[4-(oxetan-3-yl)piperazin-1-yl]pent-2-enenitrile (PRN1008) at 400 mg BID starting dose (at week 1) with intra-patient escalating dose adjustment allowed to 500 mg BID or 600 mg BID, wherein the dosing period ends at week 13;
wherein the condition for dose escalation is either (a) the patient not achieving control of disease activity (CDA) or (b) not reaching end of consolidation phase (ECP);
wherein control of disease activity is defined as the visit at which new lesions from PV or PF cease to form and established lesions from PV or PF begin to heal;
wherein end of consolidation phase is defined as the visit at which no new lesions from PV or PF have developed for a minimum of 2 weeks and a majority of established lesions from PV or PF have healed;
wherein throughout the administration of PRN1008 to the patient population, the patient population also administered a corticosteroid at a dose of less than or equal to 0.5 mg/kg/day (≤ 0.5 mg/kg/day); wherein prior to the 400 mg QD administration of PRN1008, the patient population has (a) relapsing PV; and (b) a pemphigus disease activity index (PDAI) skin score of 8-60 points, and is maintained on a low dose corticosteroid (LDCS) therapy that comprises administering a corticosteroid at a dose of ≤ 0.5 mg/kg/day.

### Pharmaceutical Compositions:

In some embodiments of the present disclosure, PRN1008 is administered as part of a pharmaceutical composition comprising: at least one compound chosen from PRN1008 and pharmaceutically acceptable salts thereof; and at least one pharmaceutically acceptable excipient. In some embodiments, the pharmaceutical composition is in the form of at least one tablet.

In some embodiments of the present disclosure, PRN1008 is orally administered to the human patient. In some embodiments of the present disclosure, PRN1008 is orally administered as part of a pharmaceutical composition comprising: at least one compound chosen from PRN1008 and pharmaceutically acceptable salts thereof; and at least one pharmaceutically acceptable excipient. In some embodiments, the pharmaceutical composition is in the form of at least one tablet.

In some embodiments of the present disclosure, PRN1008 administered to the human patient in the form of at least one tablet. In some embodiments of the present disclosure, PRN1008 is administered in the form of at least one tablet comprising: at least one compound chosen from PRN1008 and pharmaceutically acceptable salts thereof; and at least one pharmaceutically acceptable excipient.

In some embodiments, PRN1008 is administered with a glass of water.

In some embodiments, PRN1008 is administered with food.

In some embodiments, PRN1008 is administered without food.

The proportion and nature of any pharmaceutically acceptable excipient may be determined by the chosen route of administration and standard pharmaceutical practice. Except insofar as any conventional pharmaceutically acceptable excipient is incompatible with PRN1008, such as by producing any undesirable biological effect or otherwise interacting in a deleterious manner with any other component(s) of the pharmaceutically composition, its use is contemplated to be within the scope of this disclosure.

Some examples of materials which may serve as pharmaceutically acceptable excipients include: (1) sugars, such as, e.g., lactose, glucose, and sucrose; (2) starches, such as, e.g., corn starch and potato starch; (3) cellulose and its derivatives, such as, e.g., sodium carboxymethyl cellulose, ethyl cellulose, and cellulose acetate; (4) powdered tragacanth; (5) malt; (6) gelatin; (7) talc; (8) excipients, such as, e.g., cocoa butter and suppository waxes; (9) oils, such as, e.g., peanut oil, cottonseed oil, safflower oil, sesame oil, olive oil, corn oil, and soybean oil; (10) glycols, such as, e.g., propylene glycol; (11) polyols, such as, e.g., glycerin, sorbitol, mannitol, and polyethylene glycol; (12) esters, such as, e.g., ethyl oleate and ethyl laurate; (13) agar; (14) buffering agents, such as, e.g., magnesium hydroxide and aluminum hydroxide; (15) alginic acid; (16) pyrogen-free water; (17) isotonic saline; (18) Ringer's solution; (19) ethyl alcohol; (20) phosphate buffer solutions; and (21) other non-toxic compatible substances employed in pharmaceutical formulations.

Remington: The Science and Practice of Pharmacy, 21st edition, 2005, ed. D.B. Troy, Lippincott Williams & Wilkins, Philadelphia, and Encyclopedia of Pharmaceutical Technology, eds. J. Swarbrick and J. C. Boylan, 1988-1999, Marcel Dekker, New York also discloses additional examples of pharmaceutically acceptable excipients, as well as known techniques for preparing and using the same.

One skilled in the art can readily select the proper form and route of administration depending upon the disorder or condition to be treated, the stage of the disorder or condition, and other relevant circumstances.

### EXAMPLES

The following examples are intended to be illustrative and is not meant in any way to limit the scope of the disclosure.

### Abbreviations

- ABSIS: Autoimmune Bullous Skin Disorder Intensity Score
- ABQOL: Autoimmune Bullous Diseases Quality of Life (assessment)
- AE: Adverse event
- Ae: Amount excreted unchanged in the urine
- ALP: Alkaline phosphatase
- ALT: Alanine aminotransferase
- ANOVA: Analysis of variance
- AST: Aspartate aminotransferase
- AUC: Area under the plasma concentration-time curve
- BCR: B-cell receptor
- BID: Twice daily (morning and evening)
- BMI: Body mass index
- BP: Blood pressure
- BPM: Beats per minute
- BTK: Bruton's Tyrosine Kinase
- CA: Competent Authority
- CI: Confidence Interval
- CLr: Renal clearance
- Cmax: Maximum observed plasma concentration
- CNS: Clinical Network Services
- CPK: Creatine phosphokinase
- CR: Clinical Response
- CRF: Case report form
- CRO: Contract research organization
- CTCAE: Common Terminology Criteria for AEs
- CV: Coefficient of Variation
- CYP: Cytochrome P450
- DBP: Diastolic blood pressure
- DSG: Desmoglein
- EC: Ethics Committee (see also HREC)
- ECG: Electrocardiogram
- EDC: Electronic Data Capture
- ELISA: Enzyme-Linked Immunosorbent Assay
- FSH: Follicle Stimulating Hormone
- FDA: Food and Drug Administration
- GLP: Good Laboratory Practice
- H2: Histamine two (receptor)
- HBsAg: Hepatitis B surface antigen
- HPMC: Hypromellose
- HCV: Hepatitis C Virus
- HDL: High density lipoprotein
- HDPE: High-density polyethylene
- HIV: Human Immunodeficiency Virus
- HR: Heart rate
- HREC: Human Research Ethics Committee
- IB: Investigator's Brochure
- ICF: Informed Consent Form
- ICH: International Conference on Harmonization
- IMP: Investigational medicinal product
- IR: Immediate Release (tablet formulation)
- IRB: Institutional Review Board (Human Research Ethics Committee)
- IVIG: Intravenous immunoglobulin
- LDL: Low density lipoprotein
- LPLV: Last participant last visit
- LTFU: Long-term Follow-up
- MAD: Multiple ascending dose (trial)
- MedDRA: Medical Dictionary for Regulatory Activities
- N: Sample Size
- NOEL: No observed effect level
- NOAEL: No observed adverse effect level
- OTC: Over the counter
- PBMC: Peripheral Blood Mononuclear Cell
- PD: Pharmacodynamic
- PDAI: Pemphigus Disease Area Index
- PF: Pemphigus foliaceus
- PK: Pharmacokinetic
- PO: By Mouth
- PV: Pemphigus vulgaris
- Q12H: Every 12 hours
- QD: Once a day
- QTc: QT interval corrected for heart rate
- RA: Rheumatoid arthritis
- RBC: Red blood cell
- RR: Resting Rate
- SAD: Single ascending dose
- SAE: Serious adverse event
- SBP: Systolic blood pressure
- SD: Standard Deviation
- SI: Système international d'unités (International system of units)
- SMC: Safety Monitoring Committee
- SoAT: Schedule of Assessment Table
- SLE: Systemic Lupus Erythematosus
- SNAQ: Simplified Nutritional Appetite Questionnaire
- SSR: Six-Month SUSAR Report
- SUSAR: Suspected Unexpected Serious Adverse Reaction
- TABQOL: Treatment of Autoimmune Bullous Diseases Quality of Life (assessment)
- TB: Tuberculosis
- TEAE: Treatment-Emergent Adverse Event
- TGA: Therapeutic Goods Administration
- Tmax: Time of observed maximum plasma concentration
- TSH: Thyroid stimulating hormone
- t½: Elimination half-life
- WBC: White blood cell
- WHODD: World Health Organization Drug Dictionary
- XLA: X-linked agammaglobulinemia

### Example 1: Open-Label, Phase 2, Pilot Study Investigating the Safety, Clinical Activity, Pharmacokinetics, and Pharmacodynamics of Oral Treatment with the BTK Inhibitor PRN1008 in Patients with Newly Diagnosed or Relapsing Pemphigus Vulgaris

The clinical study was an open-label, phase 2 pilot cohort study investigating the safety, clinical activity, pharmacokinetics, and pharmacodynamics of oral treatment with the BTK inhibitor PRN1008 in patients with newly diagnosed or relapsing pemphigus, such as, e.g., newly diagnosed or relapsing pemphigus vulgaris. The study was conducted in accordance with ethical guidelines.

A key goal of drug development is to improve the treatment risk-benefit ratio. The current standard of care for pemphigus and other immune-mediated diseases is high-dose CS alone or in combination with other immunosuppressant drugs, which have high risk of AEs, delayed onset of action, long-term B cell depletion, and poor suitability for chronic administration. CS have limited long-term utility because the high dosages required for efficacy are associated with serious adverse events.

The primary objective of the study were: (1) to evaluate the clinical safety of PRN1008 in patients with pemphigus, such as, e.g., pemphigus vulgaris (PV), over a 12-week (Part A) or 24-week (Part B) treatment period; and (2) to evaluate the clinical activity of PRN1008 in patients with pemphigus, such as, e.g., PV, per criteria in the European Academy of Dermatology and Venereology (EADV) 2014 Pemphigus S2 Guideline (Hertl et al., 2015), wherein the definition of complete remission was modified to exclude the 2-month durability portion of the definition. A secondary objective of the study was to evaluate the pharmacokinetics (PK) and the pharmacodynamics (PD) of PRN1008 in patients with pemphigus, such as, e.g., PV. An exploratory endpoint of the study was to evaluate the relationship of PK and PD to each other and to efficacy and safety in this patient population.

**Table 1 General Dose Adjustment Guidelines for Dose Selection in the First 4 Weeks in Part A**

| **Clinical Response** | **Trough BTK Occupancy** | **Tolerability** | **Action** |
|---|---|---|---|
| Responder 400 mg *bid* | ≥ 50% | Well Tolerated | Maintain 400 mg *bid* |
| | | | Taper corticosteroids if used in combination |
| | | Poorly Tolerated | Reduce to 300 mg *bid* |
| | | | Taper corticosteroids if used in combination |
| | < 50% | Well Tolerated | Maintain 400 mg *bid* |
| | | | Taper corticosteroids if used in combination |
| | | Poorly Tolerated | Reduce to 300 mg *bid* |
| | | | Taper corticosteroids if used in combination |
| Suboptimal Response 400 mg *bid* | ≥ 50% | Well Tolerated | *Follow rescue criteria if triggered,* if not maintain dose at 400 mg *bid* |
| | | Poorly Tolerated | *Follow rescue criteria if triggered,* if not maintain dose at 400 mg *bid* if feasible |
| | < 50% | Well Tolerated | *Follow rescue criteria if triggered, if not,* increase dose to 600 mg *bid* |
| | | Poorly Tolerated | *Follow rescue criteria if triggered,* if not, increase dose to 600 mg *bid* if tolerability allows. |

In Part A (12-week treatment period study), initial PRN1008 dosing was 400 mg BID, with intra-patient dose adjustment up to 600 mg BID allowable based on BTK occupancy and clinical response, and corticosteroid rescue treatment, if indicated (Table 1). In Table 1, "well tolerated" is defined as the absence of Grade 3 or greater gastrointestinal AEs, or Grade 2 non-gastrointestinal AEs, including liver function changes, related to PRN1008 therapy. A low-dose corticosteroid (≤ 0.5 mg/kg/day of corticosteroid, wherein the corticosteroid was prednisone or an equivalent) could be administered in combination with PRN1008.

The maximum dose of PRN1008 allowable in Part A, after dose adjustment, was 600 mg BID. Patients were treated under a corticosteroid taper protocol comprising either: (1) maintaining a corticosteroid dose for 2 weeks after disease control was achieved and subsequently reducing the corticosteroid dose by 15% every three weeks; or (2) following the glucocorticoid taper schedule disclosed in Table 1 of Werth VP, et al., Arch Dematol. 2008 Jan;144(1):25-32 (hereinafter the Werth taper). Typically, subjects in Part A received twice-daily PRN1008 treatment for 12 weeks, starting on Day 1 and ending on study Day 85, with a further 12 weeks of follow up (total duration of individual subject participation is approximately 28 weeks). Typically, clinical response and tolerability were assessed at each visit.

**Table 2 General Dose Escalation Guidelines in Part B**

| **Current Dose** | **Inadequate Clinical Response Dose-Escalation Rules** |
|---|---|
| 400 mg qd | Increase to 400 mg bid (allowed at Week 3 visit or later)** |
| 400 mg bid | Increase to 600 mg bid (allowed at Week 5 visit or later)** |
| 600 mg bid | No dose increase possible. A corticosteroid rescue protocol may be initiated. |

| | |
|---|---|
| **Unless tolerability issues preclude dose-escalation | |

In Part B (24-week treatment period), the initial PRN1008 dosing was 400 mg QD, unless patients were eligible to roll from Part A to Part B, with intra-patient dose escalation to 400 mg BID allowed at or after Week 3 visit for insufficient clinical response (and then again to 600 mg BID if necessary at or after Week 5 visit) (Table 2). Inadequate clinical response in Table 2 was determined at the investigator's discretion. Generally, clinical response is shown by some improvement seen in first 2 weeks with CDA achieved by the Week 5 visit. Typically, subjects in Part B received once or twice-daily PRN1008 for 24 weeks, starting on Day 1 and ending on study Day 169, with a follow up visit 4 weeks later (a total duration of individual subject participation is approximately 32 weeks). A low-dose corticosteroid (≤ 0.5 mg/kg/day of corticosteroid, wherein the corticosteroid was prednisone or an equivalent) could be administered in combination with PRN1008. Typically, clinical response and tolerability were assessed at each visit.

### PRN1008 Initial Dosage Selection:

### 400 mg BID (Part A):

The 400 mg BID starting dose was based upon the dose known to produce ~70% BTK occupancy at trough (~85% average occupancy over the day), as adjusted by results of the relative bioavailability study, where the tablet had ~70% of the exposure of the equal dose of the liquid formulation. Adequate BTK occupancies with 400 mg BID dosing of the IR tablet have been confirmed in many patients with pemphigus studied to date. To confirm achievement of target, BTK occupancy measurements after the first dose were expeditiously processed and provided to the treating physician in time for a follow-up visit at Day 15 (Part A only). This dose level demonstrated adequate safety factors to exposures in chronic toxicology studies.

### 400 mg qd (Part B):

In some but not all animal studies, a dose-response relationship between pre-dose BTK occupancy and clinical efficacy has been observed. As it was unknown whether a once daily PRN1008 dose will provide adequate pharmacodynamic effect, a 400 mg QD dose was tested in Part B, with the option to expeditiously dose-escalate to higher doses at or after the Week 3 visit. This dose level demonstrated adequate safety factors to exposures in chronic toxicology studies.

### Maximum dose of 600 mg bid:

A dose level 50% higher than the target upper dose level of 400 mg bid was arbitrarily chosen based on previous clinical safety data in healthy volunteers at higher exposures and adequate safety factors to exposure in animal toxicology studies.

### Study Population:

The study population was comprised of male or female patients with newly diagnosed (i.e., naive to an effective induction treatment regimen) or relapsing, biopsy-proven, mild to severe PV (PDAI 8-60), for whom an initial period of PRN1008 monotherapy is judged clinically acceptable. Because patients without mucosal involvement but with a medical history suggestive of PV were allowed into the study, some patients with clinical features suggestive of the pemphigus foliaceus (PF) variant of the disease may have been enrolled.

Patients were considered to have withdrawn from the study early is they withdrew from the study before taking one or more doses of study drug.

For Part A, 52 patients were assessed for eligibility with up to 28 days of screening. 25 patients were excluded, with 6 unable to provide written consent and agree to assessment schedule, 6 positive viral (Hepatitis B and C or HIV) screening, 5 positive TB screening, 4 not within age range or not having biopsy-proven, mild-moderate PV, and 4 excluded for other reasons. 27 patients enrolled and received PRN1008 (400 mg BID - 600 mg BID), with all starting on 400 mg BID and three patients experiencing dose increases (one patient to 500 mg BID and two patients to 600 mg BID). 1 patient discontinued early due to a non-related AE (acute respiratory failure) before the primary endpoint CDA assessment. 2 patients discontinued early after the primary endpoint CDA assessment, with 2 non-related AEs (pancreatic pseudocyst; chest pain) reported between the primary endpoint CDA assessment and completion of treatment. 24 enrolled patients underwent 12-week post-treatment assessment.

For Part B, 18 patients were assessed for eligibility with up to 28 days of screening. 15 patients enrolled and received 400 mg QD with intra-patient escalating dose adjustment allowed (400 mg BID, 600 mg BID). 1 patient discontinued at week 9 due to worsening of pemphigus that started during screening after stopping MMF, which continued resulting in hospitalization at week 9.

Demographic characteristics at baseline for patients enrolled in Parts A and B are summarized in Table 3. In Table 3, moderate-severe included patients with severe, relapsing disease per PDAI severity quartiles for relapsing disease vs. mild-moderate in newly diagnosed disease.

**Table 3 Demographic Characteristics**

| **Characteristics** | | **Part A (N = 27)** | **Part B (N = 27)** |
|---|---|---|---|
| Mean age, year (SD, range) | | 52 (9, 37-72) | 46 (9.5, 30-64) |
| Gender, n (%) | Male | 12 (44) | 8 (53) |
| | Female | 15 (56) | 7 (47) |
| Pemphigus type, n (%) | Pemphigus vulgaris | 23 (85) | 13 (88) |
| | Pemphigus foliaceus | 3 (11) | 1 (7) |
| | Neither | 1 (4) | 1 (7) |
| Mean time from pemphigus diagnosis, years (mean, range) | | 6 (7, 0-25) | 1.14 (1.35, 0-5.3) |
| Mean PDAI score, points (SD, range) | | 19 (11, 8-43) | 15.5 (7.5, 8-36) |
| Disease severity, n (%) | PDAI < 15 (mild-moderate) | 11 (41) | 8 (53) |
| | PDAI ≥ 15 (moderate-severe) | 16 (59) | 7 (47) |
| Mean CS dose at entry, mg/day (SD, range) | | 14 (11, 0-30) | 21 (14, 0-50) |

### Inclusion Criteria (Part A and Part B unless noted below):

The following inclusion criteria were used to inform the enrollment of patients in this study.
1. Male or female patients, aged 18 to 80 years old, with biopsy-proven (positive direct immunofluorescence and appearance on H&E microscopy), mild-moderate PV in **Part A** (PDAI 8 to 45) and mild-severe PV in **Part B** (PDAI 8 to 60)
2. Newly diagnosed or relapsing patients for whom an initial period of PRN1008 monotherapy or combination therapy with low-dose corticosteroids (≤ 0.5 mg/kg of prednis[ol]one or equivalent), is judged clinically acceptable, provided tapering of the corticosteroid treatment regimen is anticipated with good clinical response to PRN1008
3. BMI > 17.5 and < 40 kg/m² (**Part A only**)
4. Adequate hematologic, hepatic, and renal function (absolute neutrophil count ≥ 1.5 X 10⁹/L, Hgb > 9 g/dL, platelet count ≥ 100 X 10⁹/L, AST/ALT ≤ 1.5 x ULN, albumin ≥ 3 g/dL, creatinine ≤ ULN (**Part A**) and creatinine ≤ 1.5 x ULN (**Part B**)
5. Female patients who are of reproductive potential must agree for the duration of active treatment in the study to use an effective means of contraception (hormonal contraception methods that inhibits ovulation, intrauterine device, intrauterine hormone-releasing system, bilateral tubal ligation, vasectomized partner, condoms or sexual abstinence). Unless surgically sterile, postmenopausal females should have menopause confirmed by FSH testing.
6. Able to provide written informed consent and agreeable to the schedule of assessments.

### Exclusion Criteria:

The following exclusion criteria were used to inform the enrollment of patients in this study.
1. Previous use of a BTK inhibitor
   *Patients enrolled in a previous version of the protocol who were still in their 12-week active treatment period with PRN1008 were eligible to continue treatment, initially with their current dose level, under the amended protocol for an additional 12 weeks, i.e. 24 weeks total, following review and signature of the EC approved patient's consent. Patients who completed **Part A** and did not discontinue the study due to a medical condition that might compromise safety assessments or for a PRN1008 related adverse event may be screened for entry under **Part B.***
2. Pregnant or lactating women
3. ECG findings of QTc > 450 msec (males) or > 470 msec (females), poorly controlled atrial fibrillation (*i.e.,* symptomatic patients or a ventricular rate above 100 beats/min on ECG), or other clinically significant abnormalities
4. A history of malignancy of any type, other than surgically excised non-melanoma skin cancers or in situ cervical cancer within 5 years before the day of dosing
5. Use of immunologic response modifiers with the following periods prior to Day 1: as concomitant therapy, other immunologic response modifiers not detailed in this exclusion apart from corticosteroids; *1 week:* cyclophosphamide; *4 weeks:* IVIG, Kinaret (anakinra) and Enbrel (etanercept); *12 weeks:* Remicade (infliximab), Humira (adalimumab), Simponi (golimumab), Orencia (abatercept), Actemra (tocilizumab), Cimzia (certolizumab), Cosentyx (secukinumab), plasmapheresis; *6 months:* Rituxan/MabThera (rituximab), ofatumumab, any other anti-CD20 antibody, other long-acting biologics
6. More than 0.5 mg/kg of prednis(ol)one per day ("low dose corticosteroids") within the two weeks prior to Day 1
7. Use of proton pump inhibitor drugs such as omeprazole and esomeprazole (it is acceptable to change patient to H2 receptor blocking drugs prior to the first dose of PRN1008)
8. Concomitant use of known strong-to-moderate inducers or inhibitors of CYP3A (Appendix 2) within 3 days or 5 half-lives (whichever is longer) of study drug dosing
9. Use of CYP3A-sensitive substrate drugs (Appendix 3) with a narrow therapeutic index within 3 days or 5 half-lives (whichever is longer) of study drug dosing including, but not limited to, alfentanil, astemizole, cisapride, cyclosporine, dihydroergotamine, ergotamine, fentanyl, pimozide, quinidine, sirolimus, tacrolimus, or terfenadine
10. Has received any investigational drug (or is currently using an investigational device) within the 30 days before receiving the first dose of study medication, or at least 5 times the respective elimination half-life time (whichever is longer)
11. History of drug abuse within the previous 12 months
12. Alcoholism or excessive alcohol use, defined as regular consumption of more than approximately 3 standard drinks per day
13. Refractory nausea and vomiting, malabsorption, external biliary shunt, or significant bowel resection that would preclude adequate study drug absorption
14. History of anorexia nervosa or periods of three months or more of low body weight (BMI < 17.5) in the past 5 years
15. Donation of a unit or more of blood or blood products within 4 weeks prior to Day 1
16. History of solid organ transplant
17. History of epilepsy or other forms of seizure in the last 5 years
18. Positive for screening for HIV, hepatitis B (surface and core antibodies unrelated to vaccination), or hepatitis C (anti-HCV antibody confirmed with Hep C RNA)
19. Positive interferon-gamma release assay (IGRA) (e.g., T-spot TB Test, QuantiFERON^{®}-TB Gold, or QuantiFERON^{®}-TB Gold Plus (QFT Plus) at Screening. Unless, the patient has latent TB and all of the following 3 conditions are true:
   a. Chest X-ray does not show evidence suggestive of active tuberculosis (TB) disease
   b. There are no clinical signs and symptoms of pulmonary and/or extra-pulmonary TB disease
   c. Documented receipt of one of the following prophylactic treatment regimens:
      i. Oral daily Isoniazid for 6 months
         or
      ii. Oral daily Rifampin (RIF) for 4 months
         or
      iii. Isoniazid and Rifapentine weekly for 3 months (3HP)
      On a case by case basis, after discussion and approval by the Sponsor, a local TB test that is negative and is considered equivalent to 1 of the above tests may be used for eligibility. For example, if a QuantiFERON^{®}-TB Gold, or QuantiFERON-TB Gold Plus (QFT Plus) is positive and a local blood test or T-Spot TB test is negative, the patient may be enrolled using the local result upon approval of the Sponsor.
20. History of serious infections requiring intravenous therapy with the potential for recurrence
21. Live vaccine within 28 days prior to baseline or plan to receive one during the study
22. Any other clinically significant disease, condition, or medical history that, in the opinion of the Investigator, would interfere with subject safety, study evaluations, and/or study procedures

### Prior Therapy:

Use of immunologic response modifiers within the following periods prior to Day 1 were not permitted: (1) one week for cyclophosphamide; (2) four weeks for Kinaret^{®} (anakinra), intravenous gamma globulin (IVIG), and Enbrel^{®} (etanercept); (3) 12 weeks for Remicade^{®} (infliximab), Humira^{®} (adalimumab), Simponi^{®} (golimumab), Orencia^{®} (abatercept), Actemra^{®} (tocilizumab), Cimzia^{®} (certolizumab), Cosentyx^{™} (secukinumab), plasmapheresis; and (4) 6 months for Rituxan^{®}/MabThera^{®} (rituximab), ofatumumab, any other anti-CD20 antibody, or any other long-acting biologic.

### Concomitant Therapy:

Concomitant use of immunosuppressant medication, other than low-dose corticosteroids, was avoided unless rescue criteria were triggered. Concomitant use of known strong to moderate inducers or inhibitors of CYP3A within 14 days or 5 half-lives (whichever was longer) of dosing with PRN1008 was avoided. Use of CYP3A-sensitive substrate drugs with a narrow therapeutic index within 14 days or 5 half-lives (whichever is longer) of PRN1008 dosing including, but not limited to, alfentanil, astemizole, cisapride, cyclosporine, dihydroergotamine, ergotamine, fentanyl, pimozide, quinidine, sirolimus, tacrolimus, or terfenadine was avoided. Proton pump inhibitors were not permitted.

The use of oral prednis(ol)one was considered permissible in some circumstances. For admission to the study, doses of oral prednis(ol)one in the 2 weeks prior to Day 1 could be no higher than 0.5 mg/kg per day (inhaled and mucosal [for symptomatic treatment of oral lesions] corticosteroids are allowed). Where patients entered the study on low-dose corticosteroids, the regimen could be maintained for the initial 2 weeks of PRN1008 therapy. At the Day 15 review, a good clinical response to PRN1008 could allow the tapering of the corticosteroid to commence using the Werth taper. In some circumstances, corticosteroids could be added or the dose increased, with or without cessation of PRN1008, as clinically appropriate.

### Assessments:

After providing written informed consent, subjects typically completed the Screening Assessments within 28 days before the first dose of PRN1008: (1) review of medical history and concomitant medication; (2) PDAI, ABSIS assessments; (3) review of inclusion and exclusion criteria; (4) measurement of height and weight; (5) physical examination; (6) 12-lead ECG; (7) vital signs (blood pressure, heart rate, respiration rate, and temperature); (8) clinical laboratory testing (hematology, coagulation, serum chemistry, and urinalysis) HIV, hepatitis B (surface antigen and core antigen and antibodies), hepatitis C (anti-HCV antibody confirmed with Hep C RNA); (9) TB screen with T-spot TB Test, QuantiFERON^{®}-TB Gold, or QuantiFERON^{®}-TB Gold Plus (QFT Plus); (10) serum pregnancy test for females of childbearing potential; (11) FSH (in postmenopausal women who are not surgically sterile only); (12) skin biopsy if not already performed: lesional for H&E staining, perilesional for direct immunofluorescence.

PDAI and ABSIS assessments were employed to monitor pemphigus disease activity. For most subjects in Parts A and B, abbreviated physical exams, PDAI, and ABSIS assessments were performed at the following assessments: (1) day 1, week 1 (pre-dose); (2) day 15, week 3 (± 3 days); (3) day 29, week 5 (± 3 days); (4) day 57, week 9 (± 7 days); (5) day 85, week 13 (± 7 days); (6) day 113, week 17 (± 7 days); (7) day 141, week 21 (± 7 days); (8) day 169, week 25 (± 7 days); (9) day 197, week 29 (± 7 days); and (10) any unscheduled visits. Photography was used to document skin disease changes where appropriate.

ABQOL and TABQOL assessments were employed to monitor subject quality of life. For most subjects in Parts A and B, ABQOL and TABQOL assessments were performed at the following assessments: (1) day 1, week 1 (pre-dose); (2) day 15, week 3 (± 3 days); (3) day 29, week 5 (± 3 days); (4) day 57, week 9 (± 7 days); (5) day 85, week 13 (± 7 days); (6) day 113, week 17 (± 7 days); (7) day 141, week 21 (± 7 days); (8) day 169, week 25 (± 7 days); (9) day 197, week 29 (± 7 days); and (10) any unscheduled visits.

Specific assessments to evaluate treatment safety included the following: (1) the frequency and type of AEs; (2) clinical laboratory testing; (3) SNAQ appetite questionnaire; and (4) vital signs. Typically, patients remained under observation in the clinic for 2 hours after administration of the first PRN1008 dose and until the PK sample was drawn.

### Primary Outcome Measures:

The primary safety outcome measures were incidence of treatment-emergent AEs (TEAEs), including clinically significant changes in physical examination, laboratory tests, and vital signs.

The primary efficacy outcome measures were the proportion of subjects who are able to achieve control of disease activity (CDA) within 4 weeks of starting PRN1008 treatment without the need for doses of prednis(ol)one > 0.5 mg/kg.

### Secondary Outcome Measures:

The following clinical activity endpoints were also assessed: (1) proportion of subjects able to achieve CDA without corticosteroids within 4 weeks; (2) proportion of subjects able to achieve a complete response (CR) without corticosteroids within 12 weeks (and 24 weeks in **Part B**); (3) proportion of subjects able to achieve CR without the need for doses of prednis(ol)one of greater than 0.5mg/kg within 12 weeks (and 24 weeks in **Part B**); (4) time to CDA; (5) time to CR; (6) time to end of consolidation phase; (7) time to relapse after PRN1008 treatment discontinuation; (8) cumulative corticosteroid usage over the first 12 weeks (and 24 weeks in **Part B**); (9) change from baseline in Pemphigus Disease Area Index (PDAI) and Autoimmune Bullous Skin Disorder Intensity Score (ABSIS) scores at each follow-up visit; (10) change from baseline in Autoimmune Bullous Diseases Quality of Life (ABQOL) and Treatment of Autoimmune Bullous Diseases Quality of Life (TABQOL) scores at each follow-up visit; and (11) change from baseline in appetite (SNAQ score) at each follow-up visit.

Clinical activity endpoints were as defined by the EADV 2014 pemphigus S2 guideline (Hertl et al. 2015) with the exception that CR was defined as CR at a single point in time rather than present for ≥ 2 months.

### PK/PD Measures:

PK outcome measures investigated included plasma concentrations of PRN1008 at approximately the time of maximum concentration on Day 1 and at various subsequent times during outpatient dosing. PD outcome measures investigated included percentage BTK occupancy for individuals in peripheral blood mononuclear cells (PBMCs) at 2 and 24 hours after the first PRN1008 dose and at varied subsequent times during outpatient dosing, as well as change from baseline in anti-dsg1-3 autoantibody levels by ELISA at various time points. Exploratory PK/PD analysis examined the effects, if any, of covariates on PK and/or PD, and the relationship between PK, PD, and efficacy in this population.

### Analysis Populations:

Four study populations were defined: Screening Population; Safety Population; Efficacy Population; and Pharmacokinetic Population.

All participants who provide informed consent and have screening assessments evaluated for study participation were included in the Screening Population. All participants who have received at least one dose of PRN1008 were included in the safety analysis (Safety Population). The Safety Analysis Population was defined for all safety analyses.

All patients who received at least one dose of PRN1008 were included in the efficacy analysis (Efficacy Population). Subject response and disease progression were determined using PDAI, ABSIS, ABQOL, and TABQOL scores.

The Pharmacokinetic Population included participants who provided adequate plasma concentration data to allow for PK analysis. Participants could be excluded from the PK population if they significantly violated the inclusion or exclusion criteria, deviated significantly from the protocol, or if data are unavailable or incomplete, all of which may influence the analysis.

### Clinical Adverse Events:

An adverse event (AE) is any untoward medical occurrence in a participant or clinical investigation participant administered a pharmaceutical product and which does not necessarily have to have a causal relationship with the intervention. An AE can therefore be any unfavorable and unintended sign (including an abnormal laboratory finding, for example), symptom, or disease temporally associated with the use of an investigational product, whether or not considered related to the product. Investigators were instructed to report in detail all AEs encountered during the clinical study in the source documents, from the date of participant consent throughout the follow-up visit. Investigators were also instructed to report pre-existing conditions that worsen during a study were instructed as AEs, with the exception of the disease under study as it was expected that there may be variation in pemphigus disease activity intended to be captured in other measurements.

Investigators were instructed to grade AEs based on the NCI CTCAE, Version 4.0 or higher. For any AEs not found in the CTCAE, the following intensity grading could be employed:
- Grade 1:: Mild; asymptomatic or mild symptoms; clinical or diagnostic observations only; intervention not indicated.
- Grade 2:: Moderate; minimal, local or noninvasive intervention indicated; limiting age-appropriate instrumental activities of daily living.
- Grade 3:: Severe or medically significant but not immediately life-threatening; hospitalization or prolongation of hospitalization indicated; disabling; limiting self-care activities of daily living.
- Grade 4:: Life-threatening consequences; urgent intervention indicated.
- Grade 5:: Death related to AE.

Investigators were instructed to use their knowledge of the study participant, the circumstances surrounding the event, and an evaluation of any potential alternative causes to determine whether or not an AE is considered to be related to the study drug, indicating "yes" or "no" accordingly. Investigators were asked to take the following guidance should be taken into consideration in determining if an AE was related to the study drug: (1) temporal relationship of event onset to the initiation of study drug; (2) course of the event, considering especially the effects of dose reduction, discontinuation of study drug, or reintroduction of study drug (if applicable); (3) known association of the event with the study drug or with similar treatments; (4) known association of the event with the disease under study; (5) presence of risk factors in the study participant or use of concomitant medications known to increase the occurrence of the event; and (6) presence of non-treatment-related factors that are known to be associated with the occurrence of the event

A serious adverse event (SAE) is any experience (clinical AE or abnormal laboratory test) that suggests a significant hazard, contraindication, side effect, or precaution. An SAE must fulfill at least one of the following criteria at any dose level: (1) is fatal (results in the outcome death); (2) is life-threatening; (3) requires in-patient hospitalization or prolongation of existing hospitalization; (4) results in persistent or significant disability/incapacity; (5) is a congenital anomaly/birth defect; or (6) is medically significant or requires intervention to prevent one or other of the outcomes listed above.

### Study Design Part A:

Part A was a multicenter, open-label, single arm Phase 2 study (NCT02704429) designed to evaluate the efficacy and safety of PRN1008 in pemphigus patients. Patients from 13 sites in Australia, Croatia, France, Greece, and Israel were screened for inclusion. 52 patients in Australia, Croatia, France, Greece, and Israel were screened, with 25 not meeting the eligibility criteria. The high screening failure rate was due to patients who tested positive for hepatitis B or C (n = 6) or tuberculosis (n = 5). 27 patients were enrolled and included in the safety analyses. A total of 26 patients were included in the modified ITT (mITT) population used for the primary efficacy analyses, with one patient excluded due to a non-treatment related AE. Two other patients withdrew from the study due to non-treatment related AEs, leaving a total of 24 patients who completed the study.

Mean patient age was 52.1 (range: 37-72), and the majority of patients were white (81.5%) and female (55.6%). Nine patients (33.3%) were newly diagnosed and 18 (66.7%) were relapsing. The mean time from diagnosis to screening was 6 years (range: 0-25). Eleven patients had mild to moderate pemphigus, and 16 patients had moderate to severe pemphigus. Twenty-six patients received concomitant CS medication at some point during the study. A total of 22 patients were PV phenotype (13 anti-dsg 3+, 10 anti-dsg 1/3+), three were PF phenotype (anti-dsg 1+), and one patient was double negative for anti-dsg 1/3. Median CS dose at study entry was 14 mg/day, ranging from 0-30 mg/day.

Patients received an initial dose of 400 mg PRN1008 twice daily (BID) with possible dose adjustment up to 600 mg BID at investigator discretion. Treatment duration was 12 weeks with an additional 12 weeks of follow-up off treatment. Three patients were dose escalated due to worsening of disease activity. One patient who had achieved CDA on Day 15 was increased to 500 mg bid with CS on Day 34. Two additional patients were increased to 600 mg bid; the first on Day 23 with a CS dose of 25 mg (achieved CDA on Day 29) and the second on Day 57.

Patients were permitted to receive no more than to 0.5 mg/kg/day CS in addition to the study drug regimen unless needed for disease "rescue." Patients were monitored throughout the study and assessed for vital signs, adverse events (AEs), concomitant medication, PK/PD, and other clinical laboratory tests.

The primary efficacy endpoint was the proportion of patients who achieved control of disease activity (CDA) within four weeks of starting PRN1008 treatment with a dose of CS of ≤ 0.5 mg/kg/day (Murrell DF et al., 2008). CDA was defined as the time at which new lesions ceased to form and established lesions began to heal. The primary safety endpoint was the incidence of treatment-emergent adverse events (TEAEs), including clinically significant changes in physical examination, laboratory tests, and vital signs. Secondary endpoints included PK/PD data as measured by BTK occupancy in peripheral blood mononuclear cells (PBMCs) at two and 24 hours after the first PRN1008 dose and change from baseline in anti-dsg 1 and anti-dsg 3 autoantibody levels at various time points.

Due to the small sample sizes, all p-values derived from inferential analyses were considered informative. In general, all significant testing was two-sided at significance level 0.05. All tests were made without adjustment for multiplicity or multiple comparisons. Two-sided 80% and 95% CIs of the response rate for efficacy endpoints were provided.

All patients except one had therapeutic levels of BTK occupancy in peripheral white cells (target ≥ 70%). High occupancy was achieved on Day 1 after the first dose, confirming that an adequate initial dose was used. The mean Day 1 BTK occupancy at two-hours post-dose was 88%, with a pre-dose average at steady state 87%. Rapid systemic clearance and slow off-kinetics resulted in minimal plasma levels of 9.5 ng/mL at 12 hours post-dose (trough point) with maintained high BTK occupancy of 87%.

14 of 26 (54%) patients achieved CDA with low dose CS by Day 29 (after 4 week period) (**FIG. 1**). This endpoint in the 400, 500, and 600 mg BID dosing subgroups was 12 of 23 (52%), 1 of 1 (100%), and 1 of 2 (50%), respectively. A total of three patients achieved CDA at or prior to the Week 5 visit without the use of CS (**FIG. 2**). 19 of 26 (73%) patients achieved CDA with low dose CS by Day 85 (**FIGs. 1****,** **2**).

6 of 26 (23%) patients achieved CR during the study period (**FIG. 3**). Four patients (15%) achieved CR on CS doses ≤ 0.5 mg/kg/day by Day 85, and two additional patients achieved CR on CS ≤ 0.5 mg/kg/day by Day 141. Of the patients that completed the study, 4 of 24 (16.7%) achieved CR during the treatment period, and an additional two patients (i.e., a total of 6 of 24, 25%) achieved CR on CS ≤ 0.5 mg/kg/day in the follow-up period (by Day 141). Mean CS dose was 14 mg/day (SD = 11) at baseline and 12 mg/day (SD = 10) after 12 weeks of treatment for all patients. For patients who achieved CR, the mean CS dose was 8 mg (range: 1-20 mg) at the time they achieved CR. The median CR duration was 96 days post-treatment, during which the mean CS dose was 8 mg/day (range: 0.7-20 mg/day).

A median reduction of 70% in PDAI scores and an associated reduction in anti-dsg 3 antibodies was seen over 12 weeks of treatment (**FIGs. 4****,** **5**). Reductions in PDAI scores were seen as early as two weeks into therapy in patients with moderately severe disease. In 11 patients with milder disease, PDAI scores fell in the first four weeks of therapy and all patients had PDAI scores of 5 or less on the Day 85 visit. A median reduction of autoantibody levels of up to 65% was observed, including in patients who had high levels of autoantibodies at baseline.

Results were similar across all subgroups, including new cases vs. chronic, anti-dsg 1 and 3 antibody titers <100 vs. ≥ 100, and mild vs. moderate to severe pemphigus patient groups. CDA rates in relapsing and newly diagnosed patients were 13 of 18 (72%) and 6 of 8 (75%), respectively. In patients with a positive anti-dsg 3 antibody, CDA rates were slightly higher (64%) than those in the overall mITT population.

Overall, PRN1008 rapidly improved clinical symptoms with > 50% of patients in Part A achieving CDA within four weeks and an overall median reduction in PDAI score of 70%. Efficacy across subgroups ranged from 43-64%, suggesting that treatment success is not impacted by disease characteristics and may be effective for all patients diagnosed with pemphigus. 90% of patients did not require dose escalation to achieve response. The high proportion of patients that achieved CDA by week 4 (54%), despite the lack of reduction in anti-dsg 3, suggests that this may be the result of rapid anti-inflammatory effect and independent of auto-antibody reduction. By week 12, reduction was seen in both PDAI score and in anti-dsg 3 levels. This observation may be attributed to PRN1008's three simultaneous mechanisms of action, i.e., rapid anti-inflammatory effects, neutralization of pathogenic autoantibodies, and blockade of autoantibody production.

Notably, 2/3 of the study population in Part A were patients with relapsing pemphigus who had lived with this condition for a significant amount of time (mean: 6 years) and likely had been refractory to multiple treatments. Additionally, over 50% of enrolled patients had PDAI scores consistent with moderate to severe pemphigus. Most other pemphigus treatments have only been studied in newly diagnosed patients or those who have been treated for up to 2 years (Chams-Davatchi C et al., 2013). In this study, PRN1008 demonstrated effectiveness in a population that is very difficult to treat and more representative of the real-world demographics of this condition.

PRN1008 also shows potential for reduced CS use compared to current standard of care. Patients achieved symptom improvement with low to no CS (mean dropped from 14 mg/day at baseline to 8 mg/day at CR), which compares favorably to the usual standard of care for pemphigus (typically 1 mg/kg/day or at least 60 mg/day) (Gregoriou S eta al., 2015; Cholera M et al., 2016). Three patients achieved CDA at or prior to Week 5 without CS use and four additional patients achieved CR at Week 13 without CS use. Only three patients needed dose escalation beyond the standard 400 mg bid, and only 4 patients needed CS rescue > 0.5 mg/kg in the 12 weeks off PRN1008. It is possible that longer duration of PRN1008 therapy may allow for further reduction in or even discontinuation of CS usage. If true, this would reduce the risk for CS-induced AEs that are common with current therapies and mitigate the adverse consequences of long-term, high-intensity CS therapy (Hwang JL et al., 2014; Rostaing L et al., 2016)

20 of 27 patients (74%) in the Safety Population experienced a TEAE. TEAEs reported by >10% of patients are shown in Appendix 17. AEs assessed to be related to the study drug included nausea (15%), upper abdominal pain (11%) and headache (11%). Most AEs were mild to moderate in severity (94/97 were Grade 1 or 2) and often transient.

Three patients experienced Serious AEs (SAE). The first and only treatment related SAE was cellulitis (Grade 3) on Day 26 in a patient with type II diabetes with relapsing pemphigus for nine years. After a three-day course of IV antibiotics during which the trial drug was suspended, the patient was discharged and completed all 12 weeks of treatment. The second SAE was a pancreatic pseudocyst discovered on Day 29, after which the patient withdrew from the study for elective surgery. The third SAE was acute respiratory failure (Day 8) due to inflammation of an undiagnosed congenital pulmonary sequestration. The patient did not recover and died 34 days after their last exposure to PRN1008; cause of death was judged to be brain herniation-brain artery embolism following lung surgery.

Safety results for PRN1008 in Part A indicate a favorable risk/benefit profile. The majority of TEAEs reported in this study were mild and transient, and no cases of AEs commonly associated with marketed BTKi, such as major hemorrhage, atrial fibrillation, or thrombocytopenia/neutropenia, were reported.

Study limitations for Part A include those typically associated with an open label trial design, such as the absence of a control group. Hence, the data should be interpreted carefully, and a placebo-controlled trial is needed to provide a more robust evaluation. The study duration was short, with 12 weeks of treatment and 12 weeks of follow up, and longer-term data are needed. The sample size of 27 patients was small. However, pemphigus is a relatively rare disease, and therefore, any study in this disease area would be relatively modest in size. Though approximately half of the study population was enrolled from Greece, the clinical and demographic characteristics of patients in the ITT population may be broadly representative of patients with pemphigus.

### Study Design Part B:

Part B was a multicenter, open-label, single-arm Phase 2 study (NCT02704429) designed to further evaluate the efficacy and safety of PRN1008 in pemphigus patients. Patients received an initial dose of 400 mg PRN1008 once a day (QD) with possible dose adjustment up to 600 mg BID at investigator discretion. Treatment duration was 24 weeks with an additional 4 weeks of follow-up off treatment. One patient dropped out due to worsening of pemphigus at week 5.

In Part B, treatment with PRN1008 delivered high CDA rates (**FIGs. 6****,** **7****,** **8A, 8B**). Patients on 400 mg QD dosing were able to achieve CDA at 4 weeks with a low dose corticosteroid but at lower rates compared to BID dosing. The overall CDA rate at 12 weeks was 80% (12 out of 15 patients).

Patients on 400 mg QD dosing had lower rates of complete remission at 12 weeks (**FIGs. 9A, 9B**) compared to BID dosing. However, by 24 weeks, a 94% reduction in median PDAI activity score and a 79% reduction in mean PDAI activity score were observed for patients enrolled in Part B (**FIGs. 10****,** **11**). Median PDAI went from 12 at day 1 to 4 at 12 weeks and 1 at 24 weeks, with 10 out of 15 patients (67%) having reached a PDAI of 1 or 0 at 24 weeks.

In addition, 24 weeks of PRN1008 treatment decreased daily corticosteroid usage (**FIG. 10**). While the cumulative steroid dose increased between the first 12 weeks and the second 12 weeks in Part A, the opposite was true in Part B.

Overall, PRN1008 was well tolerated in Part B and continued to support a positive benefit/risk for pemphigus patients. All treatment-related AEs were mild-moderate and transient. Part B treatment-related adverse events were consistent with those observed in Part A, with most common AEs being gastrointestinal in origin. In Part B, 2 of 15 patients reported a mild related infection (1 event each: grade 1 nasopharyngitis; grade 1 tracheitis). Treatment-related AEs with an incidence greater than 10% were nausea, abdominal distension, infection, and oropharyngeal pain that were mild to moderate (grade 1 and 2).

Certain documents are referred to in this application in short citation format. More detailed citations for referenced documents are provided below.

Byrd JC, Furman RR, Coutre SE, Flinn IW, Burger JA, Blum KA, Grant B, Sharman JP, Coleman M, Wierda WG, Jones JA, Zhao W, Heerema NA, Johnson AJ, Sukbuntherng J, Chang BY, Clow F, Hedrick E, Buggy JJ, James DF, O'Brien S. Targeting BTK with Ibrutinib in Relapsed Chronic Lymphocytic Leukemia. N Engl J Med., 369(1):32-42, 2013.

Bizikova P, Olivry T, Mamo LB, Dunston SM. Serum autoantibody profiles of IgA, IgE and IgM in canine pemphigus foliaceus. Vet Dermatol 2014;25:471-e75.

Bizikova P, Dean GA, Hashimoto T, Olivry T. Cloning and establishment of canine desmocollin-1 as a major autoantigen in canine pemphigus foliaceus. Vet Immunol Immunopathol 2012;149:197-207.

Development Report #DVR0210, A Pilot Study of the Efficacy of a Bruton's Tyrosine Kinase Inhibitor (BTKi) in the Treatment of Dogs with Pemphigus Foliaceus (PF). Principia Biopharma, Inc., South San Francisco, CA, 94080, U.S.A.

Evans EK, Tester R, Aslanian S, Karp R, Sheets M, Labenski MT, Witowski SR, Lounsbury H, Chaturvedi P, Mazdiyasni H, Zhu Z, Nacht M, Freed MI, Petter RC, Dubrovskiy A, Singh J, Westlin WF. Inhibition of Btk with CC-292 Provides Early Pharmacodynamic Assessment of Activity in Mice and Humans. J Pharmacol Exp Ther, 346(2):219-28, 2013.

Hertl, M., Jedlickova, H., Karpati, S., et al. (2015), Pemphigus. S2 Guideline for diagnosis and treatment - guided by the European Dermatology Forum (EDF) in cooperation with the European Academy of Dermatology and Venereology (EADV). Journal of the European Academy of Dermatology and Venereology, 29: 405-414. doi: 10.1111/jdv.12772.

Horváth, B., Huizinga, J., Pas, H.H., Mulder, A.B., Jonkman, M.F., Low Dose rituximab is effective in pemphigus. Br J Dermatol. 166(2): 405-12, 2012.

Imbruvica [package insert]. Pharmacyclics, Inc., Sunnyvale, CA; 2015.

Murrell DF, Dick S, Ahmed AR, Amagai M, Barnadas MA, Borradori L, et al. Consensus statement on definitions of disease, end points, and therapeutic response for pemphigus. J Am Acad Dermatol. 2008;58:1043-6.

Mohamed AJ, Yu L, Backesjo CM, Vargas L, Faryal R, et al. Bruton's tyrosine kinase (Btk): function, regulation, and transformation with special emphasis on the PH domain. Immunol Rev 228, 58-73, 2009.

PRN1008-005 Interim Analysis Report February 2017

PRN1008 Investigator Brochure, Principia Biopharma.

Principia Study PRN1008-006, data on file, 2016.

Rosenbach M, Murrell D, Bystryn JC, et al. Reliability and convergent validity of two outcome instruments for pemphigus. J Invest Dermatol 2009;129(10):2404-10.

Sideras P and Smith CI. Molecular and cellular aspects of X-linked agammaglobulinemia. Adv Immunol, 59: 135-223, 1995.

Tjokrowidjaja A, Daniel BS, Frew JW, Sebaratnam DF, Hanna AM, Chee S, Dermawan A, Wang CQ, Lim C, Venugopal SS, Rhodes LM, Welsh B, Nijsten T, Murrell DF. Br J Dermatol. 2013 Nov;169(5):1000-6.

Tsukada, S., Saffran, D. C., Rawlings, D. J., Parolini, O., Allen, R. C., Klisak, I., Sparkes, R. S., Kubagawa, H., Mohandas, T., Quan, S., and et al. Deficient expression of a B cell cytoplasmic tyrosine kinase in human X-linked agammaglobulinemia. Cell, 72: 279-290, 1993.

Drug Development and Drug Interactions: Table of Substrates, Inhibitors and Inducers. U.S. Food and Drug Administration. http://www.fda.gov/drugs/developmentapprovalprocess/developmentresources/druginteract ionslabeling/ucm093664.htm# Accessed 07 April 2016.

Vetrie, D., Vorechovsky, I., Sideras, P., Holland, J., Davies, A., Flinter, F., Hammarstrom, L., Kinnon, C., Levinsky, R., Bobrow, M., and et al. The gene involved in X-linked agammaglobulinaemia is a member of the src family of protein-tyrosine kinases. Nature, 361: 226-233, 1993.

Wang ML, Rule S, Martin P, Goy A, Auer R, Kahl BS, Jurczak W, Advani RH, Romaguera JE, Williams ME, Barrientos JC, Chmielowska E, Radford J, Stilgenbauer S, Dreyling M, Jedrzejczak WW, Johnson P, Spurgeon SE, Li L, Zhang L, Newberry K, Ou Z, Cheng N, Fang B, McGreivy J, Clow F, Buggy JJ, Chang BY, Beaupre DM, Kunkel LA, Blum KA. Targeting BTK with Ibrutinib in Relapsed or Refractory Mantle-Cell Lymphoma. N Engl J Med. 2013 Jun 19. [Epub ahead of print].

Werth VP, Fivenson D, Pandya AG, et al. Multicenter randomized, double-blind, placebo-controlled, clinical trial of dapsone as a glucocorticoid-sparing agent in maintenance-phase pemphigus vulgaris. Arch Dematol. 2008 Jan;144(1):25-32.

Wilson MM, Thomas DR, Rubenstein LZ, Chibnall JT, Anderson S, Baxi A, Diebold MR, Morley JE. Appetite assessment: simple appetite questionnaire predicts weight loss in community-dwelling adults and nursing home residents. Am J Clin Nutr. 2005 Nov;82(5):1074-81.

Scully C, Challacombe SJ. Pemphigus vulgaris: update on etiopathogenesis, oral manifestations, and management. Crit Rev Oral Biol Med 2002;13:397-408.

Scully C, Paes De Almeida O, Porter SR, Gilkes JJ. Pemphigus vulgaris: the manifestations and long-term management of 55 patients with oral lesions. Br J Dermatol 1999;140:84-9.

Amagai M, Stanley JR. Desmoglein as a target in skin disease and beyond. J Invest Dermatol 2012;132:776-84.

Diaz LA, Giudice GJ. End of the century overview of skin blisters. Arch Dermatol 2000;136:106-12.

Murrell DF, Peña S, Joly P, et al. Diagnosis and Management of Pemphigus: recommendations by an International Panel of Experts. Journal of the American Academy of Dermatology 2018.

Kasperkiewicz M, Ellebrecht CT, Takahashi H, et al. Pemphigus. Nat Rev Dis Primers 2017;3:17026.

Joly P, Maho-Vaillant M, Prost-Squarcioni C, et al. First-line rituximab combined with short-term prednisone versus prednisone alone for the treatment of pemphigus (Ritux 3): a prospective, multicentre, parallel-group, open-label randomised trial. Lancet 2017;389:2031-40.

RITUXAN (rituximab) Highlights of Prescribing Information. 2018. at https://www.accessdata.fda.gov/drugsatfda_docs/label/2018/103705s5450lbl.pdf.)

Cianchini G, Corona R, Frezzolini A, Ruffelli M, Didona B, Puddu P. Treatment of Severe Pemphigus With Rituximab: Report of 12 Cases and a Review of the Literature. JAMA Dermatology 2007;143:1033-8.

Harman KE, Brown D, Exton LS, et al. British Association of Dermatologists' guidelines for the management of pemphigus vulgaris 2017. Br J Dermatol 2017;177:1170-201.

Amagai M, Ikeda S, Shimizu H, et al. A randomized double-blind trial of intravenous immunoglobulin for pemphigus. J Am Acad Dermatol 2009;60:595-603.

Martin LK, Werth V, Villanueva E, Segall J, Murrell DF. Interventions for pemphigus vulgaris and pemphigus foliaceus. Cochrane Database Syst Rev 2009:Cd006263.

Crofford LJ, Nyhoff LE, Sheehan JH, Kendall PL. The role of Bruton's tyrosine kinase in autoimmunity and implications for therapy. Expert Rev Clin Immunol 2016;12:763-73.

Pal Singh S, Dammeijer F, Hendriks RW. Role of Bruton's tyrosine kinase in B cells and malignancies. Mol Cancer 2018;17:57.

Volmering S, Block H, Boras M, Lowell CA, Zarbock A. The Neutrophil Btk Signalosome Regulates Integrin Activation during Sterile Inflammation. Immunity 2016;44:73-87.

Khan WN, Alt FW, Gerstein RM, et al. Defective B cell development and function in Btk-deficient mice. Immunity 1995;3:283-99.

Montalban X, Arnold DL, Weber MS, et al. Placebo-Controlled Trial of an Oral BTK Inhibitor in Multiple Sclerosis. N Engl J Med 2019;380:2406-17.

Norman P. Investigational Bruton's tyrosine kinase inhibitors for the treatment of rheumatoid arthritis. Expert Opin Investig Drugs 2016;25:891-9.

Tam CS, LeBlond V, Novotny W, et al. A head-to-head Phase III study comparing zanubrutinib versus ibrutinib in patients with Waldenstrom macroglobulinemia. Future Oncol 2018;14:2229-37.

Crawford JJ, Johnson AR, Misner DL, et al. Discovery of GDC-0853: A Potent, Selective, and Noncovalent Bruton's Tyrosine Kinase Inhibitor in Early Clinical Development. J Med Chem 2018;61:2227-45.

Min TK, Saini SS. Emerging Therapies in Chronic Spontaneous Urticaria. Allergy Asthma Immunol Res 2019;11:470-81.

Gillooly KM, Pulicicchio C, Pattoli MA, et al. Bruton's tyrosine kinase inhibitor BMS-986142 in experimental models of rheumatoid arthritis enhances efficacy of agents representing clinical standard-of-care. PLoS One 2017;12:e0181782.

Nadeem A, Ahmad SF, Al-Harbi NO, et al. Inhibition of Bruton's tyrosine kinase and IL-2 inducible T-cell kinase suppresses both neutrophilic and eosinophilic airway inflammation in a cockroach allergen extract-induced mixed granulocytic mouse model of asthma using preventative and therapeutic strategy. Pharmacol Res 2019;148:104441

Drug Record Kinase Inhibitors. In: Services NIoHUSDoHH, ed.2019.

Khan Y, O'Brien S. Acalabrutinib and its use in treatment of chronic lymphocytic leukemia. Future Oncol 2019;15:579-89.

Paydas S. Management of adverse effects/toxicity of ibrutinib. Crit Rev Oncol Hematol 2019;136:56-63.

IMBRUVICA (ibrutinib) Highlights of Prescribing Information. US Food and Drug Administration, 2013. at https://www.accessdata.fda.gov/drugsatfda_docs/label/2015/205552s002lbl.pdf.)

Rigg RA, Aslan JE, Healy LD, et al. Oral administration of Bruton's tyrosine kinase inhibitors impairs GPVI-mediated platelet function. Am J Physiol Cell Physiol 2016;310:C373-80.

Tang CPS, McMullen J, Tam C. Cardiac side effects of bruton tyrosine kinase (BTK) inhibitors. Leuk Lymphoma 2018;59:1554-64.

Smith PF, Krishnarajah J, Nunn PA, et al. A phase I trial of PRN1008, a novel reversible covalent inhibitor of Bruton's tyrosine kinase, in healthy volunteers. Br J Clin Pharmacol 2017;83:2367-76.

Serafimova IM, Pufall MA, Krishnan S, et al. Reversible targeting of noncatalytic cysteines with chemically tuned electrophiles. Nat Chem Biol 2012;8:471-6.

Hill R BJ, Bisconte A, Tam D, Owens T, Brameld K, et al.. Preclinical Characterization of PRN1008, a Novel Reversible Covalent Inhibitor of BTK that Shows Efficacy in a RAT Model of Collagen-Induced Arthritis.. EULAR. Rome2015.

Smith PF K, Nunn PA, Hill RJ, Karr D, Tam D, et al.. A Phase 1 Clinical Trial of PRN1008, An Oral, Reversible, Covalent BTK Inhibitor Demonstrates Clinical Safety and Therapeutic Levels of BTK Occupancy Without Sustained Systemic Exposure. EULAR 2015. Rome2015.

Langrish C BJ, Francesco M, Xing Y, Shu J, LaStant J, Owens T, Brameld K, Outerbridge C, Bisconte A, White S, Hill R, Gourlay S, Goldestein D, Nunn P Pre-clinical data on the anti-inflammatory and autoantibody inhibitory effects of the reversible, covalent Bruton's tyrosin kinase inhibitor PRN1008 for autoimmune diseases [IN PRESS]. Journal of Immunology 2019.

Langrish CL, Bradshaw JM, Owens TD, et al. PRN1008, a Reversible Covalent BTK Inhibitor in Clinical Development for Immune Thrombocytopenic Purpura. Blood 2017;130:1052-.

Hill R BJ, Bisconte A, Tam D, Owens T, Brameld K, Smith P, Funk J, Goldstein D, Nunn P Preclinical Characterization of PRN1008, a Novel Reversible Covalent Inhibitor of BTK That Shows Efficacy in a Rat Model of Collagen-Induced Arthritis. The Annual European Congress of Rheumatology EULAR. Rome, Italy: EULAR; 2015.

Murrell DF ea. Final Results of the Believe-PV Proof of Concept Study of PRN1008 in Pemphigus. American Academy of Dermatology Annual Meeting. Washington DC2019.

CALQUENCE (acalabrutinib) Highlights of Prescribing Information. US Food and Drug Administration, 2017. at https://www.accessdata.fda.gov/drugsatfda_docs/label/2017/210259s000lbl.pdf.)

Boulard C, Duvert Lehembre S, Picard-Dahan C, et al. Calculation of cut-off values based on the Autoimmune Bullous Skin Disorder Intensity Score (ABSIS) and Pemphigus Disease Area Index (PDAI) pemphigus scoring systems for defining moderate, significant and extensive types of pemphigus. Br J Dermatol 2016;175:142-9.

Murrell DF, Dick S, Ahmed AR, et al. Consensus statement on definitions of disease, end points, and therapeutic response for pemphigus. J Am Acad Dermatol 2008;58:1043-6.

Anhalt GJ, Diaz LA. Research Advances in Pemphigus. JAMA 2001;285:652-4.

Chams-Davatchi C, Mortazavizadeh A, Daneshpazhooh M, et al. Randomized double blind trial of prednisolone and azathioprine, vs. prednisolone and placebo, in the treatment of pemphigus vulgaris. J Eur Acad Dermatol Venereol 2013;27:1285-92.

Gregoriou S, Efthymiou O, Stefanaki C, Rigopoulos D. Management of pemphigus vulgaris: challenges and solutions. Clin Cosmet Investig Dermatol 2015;8:521-7.

Cholera M, Chainani-Wu N. Management of Pemphigus Vulgaris. Adv Ther 2016;33:910-58.

Hwang JL, Weiss RE. Steroid-induced diabetes: a clinical and molecular approach to understanding and treatment. Diabetes Metab Res Rev 2014;30:96-102.

Rostaing L, Malvezzi P. Steroid-Based Therapy and Risk of Infectious Complications. PLoS Med 2016;13:e1002025.

Claims or descriptions that include "or" or "and/or" between at least one members of a group are considered satisfied if one, more than one, or all of the group members are present in, employed in, or otherwise relevant to a given product or process unless indicated to the contrary or otherwise evident from the context. The disclosure includes embodiments in which exactly one member of the group is present in, employed in, or otherwise relevant to a given product or process. The disclosure includes embodiments in which more than one, or all the group members are present in, employed in, or otherwise relevant to a given product or process.

Where ranges are given, endpoints are included. Furthermore, unless otherwise indicated or otherwise evident from the context and understanding of one of ordinary skill in the art, values that are expressed as ranges can assume any specific value or sub-range within the stated ranges in different embodiments of the disclosure, to the tenth of the unit of the lower limit of the range, unless the context clearly dictates otherwise.

The foregoing disclosure has been described in some detail by way of illustration and example, for purposes of clarity and understanding. Therefore, it is to be understood that the above description is intended to be illustrative and not restrictive.

## Claims

1. A compound which is (R)-2-[3-[4-amino-3-(2-fluoro-4-phenoxy-phenyl)pyrazolo[3,4-d]pyrimidin-1-yl]piperidine-1-carbonyl]-4-methyl-4-[4-(oxetan-3-yl)piperazin-1-yl]pent-2-enenitrile or a pharmaceutically acceptable salt thereof, for use in treating pemphigus, wherein said compound is administered to a human patient twice a day at a dose of 400 mg for at least 14 days, and wherein the human patient is **characterized by** relapsing pemphigus prior to the administration of said compound.

2. The compound for use of claim 1, wherein said compound is administered to the human patient at a dose of 400 mg of said compound twice a day for 14 to 84 days.

3. The compound for use of claim 1 or 2, wherein said compound is administered to the human patient in combination with a first corticosteroid at a dose of less than or equal to 0.5 mg/kg/day.

4. The compound for use of claim 3, wherein the first corticosteroid is chosen from prednisone, prednisolone, and methylprednisolone.

5. The compound for use of any one of claims 1 to 4, wherein the human patient is **characterized by** a pemphigus disease activity index (PDAI) skin score from 8 points to 60 points prior to the administration of said compound.

6. The compound for use of any one of claims 1 to 5, wherein the human patient is administered a maintenance dose of less than or equal to 0.5 mg/kg/day of a second corticosteroid prior to the administration of said compound.

7. The compound for use of any one of claims 1 to 6, wherein said compound comprises the (E) isomer of (R)-2-[3-[4-amino-3-(2-fluoro-4-phenoxy-phenyl)pyrazolo[3,4-d]pyrimidin-1-yl]piperidine-1-carbonyl]-4-methyl-4-[4-(oxetan-3-yl)piperazin-1-yl]pent-2-enenitrile.

8. The compound for use of any one of claims 1 to 6, wherein said compound comprises the (Z) isomer of (R)-2-[3-[4-amino-3-(2-fluoro-4-phenoxy-phenyl)pyrazolo[3,4-d]pyrimidin-1-yl]piperidine-1-carbonyl]-4-methyl-4-[4-(oxetan-3-yl)piperazin-1-yl]pent-2-enenitrile.

9. The compound for use of any one of claims 1 to 6, wherein said compound comprises a mixture of (E) and (Z) isomers of (R)-2-[3-[4-amino-3-(2-fluoro-4-phenoxyphenyl)pyrazolo[3,4-d]pyrimidin-1-yl]piperidine-1-carbonyl]-4-methyl-4-[4-(oxetan-3-yl)piperazin-1-yl]pent-2-enenitrile.

10. The compound for use of any one of claims 1 to 9, wherein it is for treating pemphigus vulgaris.

11. The compound for use of any one of claims 1 to 9, wherein it is for treating pemphigus foliaceus.

12. The compound for use of any one of claims 1 to 11, wherein it is for reducing average daily corticosteroid usage by the human patient.

13. The compound for use of any one of claims 1 to 12, wherein it is for healing at least 50% of established pemphigus lesions.

14. The compound for use of any one of claims 1 to 13, wherein it is for preventing new pemphigus lesion formation.

15. The compound for use of any one of claims 1 to 14, wherein it is for reducing a pemphigus disease activity index skin score, preferably for reducing said index by at least 20%.

16. The compound for use of any one of claims 1 to 15, wherein the human patient is **characterized by** a pemphigus disease activity index skin score of 0 or 1 following the administration of said compound.

17. The compound for use of any one of claims 1 to 16, wherein it further comprises achieving a control of disease activity of pemphigus.

18. The compound for use of any one of claims 1 to 16, wherein it further comprises achieving an end of consolidation phase of pemphigus.

19. The compound for use of any one of claims 1 to 17, wherein said compound is orally administered to the human patient.

## Patentansprüche

1. Verbindung, die (R)-2-[3-[4-Amino-3-(2-fluor-4-phenoxy-phenyl)pyrazolo[3,4-d]pyrimidin-1-yl]piperidin-1-carbonyl]-4-methyl-4-[4-(oxetan-3 -yl)piperazin-1-yl]pent-2-ennitril oder ein pharmazeutisch annehmbares Salz davon ist, zur Verwendung bei der Behandlung von Pemphigus, wobei die Verbindung einem menschlichen Patienten zweimal täglich in einer Dosis von 400 mg für mindestens 14 Tage verabreicht wird und wobei der menschliche Patient vor der Verabreichung der Verbindung durch einen rezidivierenden Pemphigus gekennzeichnet ist.

2. Verbindung zur Verwendung nach Anspruch 1, wobei die Verbindung dem menschlichen Patienten in einer Dosis von 400 mg der Verbindung zweimal täglich für 14 bis 84 Tage verabreicht wird.

3. Verbindung zur Verwendung nach Anspruch 1 oder 2, wobei die Verbindung dem menschlichen Patienten in Kombination mit einem ersten Kortikosteroid in einer Dosis von weniger als oder gleich 0,5 mg/kg/Tag verabreicht wird.

4. Verbindung zur Verwendung nach Anspruch 3, wobei das erste Kortikosteroid aus Prednison, Prednisolon und Methylprednisolon ausgewählt ist.

5. Verbindung zur Verwendung nach einem der Ansprüche 1 bis 4, wobei der menschliche Patient vor der Verabreichung der Verbindung durch einen Pemphigus-Erkrankungsaktivitäts-Index(PDAI)-Hautscore von 8 Punkten bis 60 Punkten gekennzeichnet ist.

6. Verbindung zur Verwendung nach einem der Ansprüche 1 bis 5, wobei dem menschlichen Patienten vor der Verabreichung der Verbindung eine Erhaltungsdosis von weniger als oder gleich 0,5 mg/kg/Tag eines zweiten Kortikosteroids verabreicht wird.

7. Verbindung zur Verwendung nach einem der Ansprüche 1 bis 6, wobei die Verbindung das (E)-Isomer von (R)-2-[3-[4-Amino-3-(2-fluor-4-phenoxy-phenyl)pyrazolo[3,4-d]pyrimidin-1-yl]piperidin-1-carbonyl]-4-methyl-4-[4-(oxetan-3-yl)piperazin-1-yl]pent-2-ennitril umfasst.

8. Verbindung zur Verwendung nach einem der Ansprüche 1 bis 6, wobei die Verbindung das (Z)-Isomer von (R)-2-[3-[4-Amino-3-(2-fluor-4-phenoxy-phenyl)pyrazolo[3,4-d]pyrimidin-1-yl]piperidin-1-carbonyl]-4-methyl-4-[4-(oxetan-3-yl)piperazin-1-yl]pent-2-ennitrile umfasst.

9. Verbindung zur Verwendung nach einem der Ansprüche 1 bis 6, wobei die Verbindung eine Mischung aus (E)- und (Z)-Isomeren von (R)-2-[3-[4-Amino-3-(2-fluor-4-phenoxyphenyl)pyrazolo[3,4-d]pyrimidin-1-yl]piperidin-1-carbonyl]-4-methyl-4-[4-(oxetan-3-yl)piperazin-1-yl]pent-2-ennitrile umfasst.

10. Verbindung zur Verwendung nach einem der Ansprüche 1 bis 9, wobei sie zur Behandlung von Pemphigus vulgaris bestimmt ist.

11. Verbindung zur Verwendung nach einem der Ansprüche 1 bis 9, wobei sie zur Behandlung von Pemphigus foliaceus bestimmt ist.

12. Verbindung zur Verwendung nach einem der Ansprüche 1 bis 11, wobei sie zur Verringerung der durchschnittlichen täglichen Kortikosteroid-Einnahme durch den menschlichen Patienten bestimmt ist.

13. Verbindung zur Verwendung nach einem der Ansprüche 1 bis 12, wobei sie zur Heilung von mindestens 50 % der bestehenden Pemphigus-Läsionen bestimmt ist.

14. Verbindung zur Verwendung nach einem der Ansprüche 1 bis 13, wobei sie zur Verhinderung der Bildung neuer Pemphigus-Läsionen bestimmt ist.

15. Verbindung zur Verwendung nach einem der Ansprüche 1 bis 14, wobei sie zur Verringerung eines Pemphigus-Erkrankungsaktivitäts-Index-Hautscores bestimmt ist, vorzugsweise zur Verringerung des Index um mindestens 20 %.

16. Verbindung zur Verwendung nach einem der Ansprüche 1 bis 15, wobei der menschliche Patient nach der Verabreichung der Verbindung durch einen Pemphigus-Erkrankungsaktivitäts-Index-Hautscore von 0 oder 1 gekennzeichnet ist.

17. Verbindung zur Verwendung nach einem der Ansprüche 1 bis 16, wobei sie ferner das Erreichen einer Kontrolle der Pemphigus-Krankheitsaktivität umfasst.

18. Verbindung zur Verwendung nach einem der Ansprüche 1 bis 16, wobei sie ferner das Erreichen eines Endes der Konsolidierungsphase von Pemphigus umfasst.

19. Verbindung zur Verwendung nach einem der Ansprüche 1 bis 17, wobei die Verbindung dem menschlichen Patienten oral verabreicht wird.

## Revendications

1. Composé, qui est (R)-2-[3-[4-amino-3-(2-fluoro-4-phénoxy-phényl)pyrazolo[3,4-d]pyrimidin-1-yl]pipéridine-1-carbonyl]-4-méthyl-4-[4-(oxétan-3-yl)pipérazin-1-yl]pent-2-ènenitrile, ou sel pharmaceutiquement acceptable de celui-ci, destiné à être utilisé dans le traitement de pemphigus, dans lequel ledit composé est administré à un patient humain deux fois par jour à une dose de 400 mg pendant au moins 14 jours, et dans lequel le patient humain est **caractérisé par** un pemphigus récidivant avant l'administration dudit composé.

2. Composé destiné à être utilisé de la revendication 1, dans lequel ledit composé est administré au patient humain à une dose de 400 mg dudit composé deux fois par jour pendant 14 à 84 jours.

3. Composé destiné à être utilisé de la revendication 1 ou 2, dans lequel ledit composé est administré au patient humain en association avec un premier corticostéroïde à une dose inférieure ou égale à 0,5 mg/kg/jour.

4. Composé destiné à être utilisé de la revendication 3, dans lequel le premier corticostéroïde est choisi parmi la prednisone, la prednisolone, et la méthylprednisolone.

5. Composé destiné à être utilisé de l'une quelconque des revendications 1 à 4, dans lequel le patient humain est **caractérisé par** un score cutané d'indice d'activité de la maladie du pemphigus (Pemphigus Disease Activity Index, PDAI) de 8 points à 60 points avant l'administration dudit composé.

6. Composé destiné à être utilisé de l'une quelconque des revendications 1 à 5, dans lequel une dose de maintien d'un second corticostéroïde inférieure ou égale à 0,5 mg/kg/jour est administrée au patient humain avant l'administration dudit composé.

7. Composé destiné à être utilisé de l'une quelconque des revendications 1 à 6, dans lequel ledit composé comprend l'isomère (E) de (R)-2-[3-[4-amino-3-(2-fluoro-4-phénoxy-phényl)pyrazolo[3,4-d]pyrimidin-1-yl]pipéridine-1-carbonyl]-4-méthyl-4-[4-(oxétan-3-yl)pipérazin-1-yl]pent-2-ènenitrile.

8. Composé destiné à être utilisé de l'une quelconque des revendications 1 à 6, dans lequel ledit composé comprend l'isomère (Z) de (R)-2-[3-[4-amino-3-(2-fluoro-4-phénoxy-phényl)pyrazolo[3,4-d]pyrimidin-1-yl]pipéridine-1-carbonyl]-4-méthyl-4-[4-(oxétan-3-yl)pipérazin-1-yl]pent-2-ènenitrile.

9. Composé destiné à être utilisé de l'une quelconque des revendications 1 à 6, dans lequel ledit composé comprend un mélange d'isomères (E) et (Z) de (R)-2-[3-[4-amino-3-(2-fluoro-4-phénoxyphényl)pyrazolo[3,4-d]pyrimidin-1-yl]pipéridine-1-carbonyl]-4-méthyl-4-[4-(oxétan-3-yl)pipérazin-1-yl]pent-2-ènenitrile.

10. Composé destiné à être utilisé de l'une quelconque des revendications 1 à 9, dans lequel il est destiné à traiter le pemphigus vulgaire.

11. Composé destiné à être utilisé de l'une quelconque des revendications 1 à 9, dans lequel il est destiné à traiter le pemphigus foliacé.

12. Composé destiné à être utilisé de l'une quelconque des revendications 1 à 11, dans lequel il est destiné à réduire l'utilisation journalière moyenne du corticostéroïde par le patient humain.

13. Composé destiné à être utilisé de l'une quelconque des revendications 1 à 12, dans lequel il est destiné à guérir au moins 50 % des lésions établies dues au pemphigus.

14. Composé destiné à être utilisé de l'une quelconque des revendications 1 à 13, dans lequel il est destiné à empêcher la formation de nouvelles lésions dues au pemphigus.

15. Composé destiné à être utilisé de l'une quelconque des revendications 1 à 14, dans lequel il est destiné à réduire un score cutané d'indice d'activité de la maladie du pemphigus, de préférence à réduire ledit indice d'au moins 20 %.

16. Composé destiné à être utilisé de l'une quelconque des revendications 1 à 15, dans lequel le patient humain est **caractérisé par** un score cutané d'indice d'activité de la maladie du pemphigus de 0 ou 1 suivant l'administration dudit composé.

17. Composé destiné à être utilisé de l'une quelconque des revendications 1 à 16, dans lequel il permet en outre d'obtenir un contrôle de l'activité de la maladie du pemphigus.

18. Composé destiné à être utilisé de l'une quelconque des revendications 1 à 16, dans lequel il permet en outre d'obtenir une fin de la phase de consolidation du pemphigus.

19. Composé destiné à être utilisé de l'une quelconque des revendications 1 à 17, dans lequel ledit composé est administré oralement au patient humain.
